# EUROPEAN PATENT SPECIFICATION

(11) **EP 0 448 511 B1**
(45) Date of publication and mention of the grant of the patent: **18.07.2001**
(21) Application number: 91810144.5
(22) Date of filing: 04.03.1991
(51) Int. Cl.: A01N 63/00, A01H 5/00, C12N 15/82

(54) **Anti-pathogenically effective compositions comprising lytic peptides and hydrolytic enzymes**
Lytische Peptide und hydrolytische Enzyme enthaltende antipathogen wirksame Zusammensetzungen
Combinaisons antipathogènes contenant les peptides lytiques et les enzymes hydrolytiques

(30) Priority: 12.03.1990 US 491801
(43) Date of publication of application: 25.09.1991
(60) Divisional application: 97117612.8
(73) Proprietor: Syngenta Participations AG, 4058 Basel (CH)
(72) Inventor: Ryals, John A., Durham, N.C. 27713 (US); Gay, Philippe Bernard, Dr., F-68100 Mulhouse (FR); Ahl Goy, Patricia A., Dr., CH-4054 Basle (CH); Garcia-Olmedo, Francisco, Prof., E-28016 Madrid (ES)
(74) Representative: VOSSIUS & PARTNER

(56) References cited:
- EP-A- 0 374 823
- WO-A-88/00976
- WO-A-89/04371
- DERWENT CENTRAL PATENTS INDEX, BASIC ABSTRACTS JOURNAL, week B29, 17th September 1979, section C: AGDOC, abstract no. J54073-182, Derwent Publications Ltd, London, GB; & JP-A-77 139 423 (MITSUI PETROCHEM. IND. K.K.) 12-06-1979
- CHEMICAL ABSTRACTS, vol. 87, no. 21, 21st November 1977, page 94, abstract no. 162156d, Columbus, Ohio, US; P.M. MILLER et al.: "Effects of hydrolytic enzymes on plant-parasitic nematodes", & J. NEMATOL. 1977, 9(3), 192-7

## Description

The present invention relates to compositions for controlling plant pathogens by employing as active ingredients a combination of lytic peptides and hydrolytic enzymes. More particularly, this invention relates to anti-pathogenic compositions in which small amounts of chitinase and/or β-1,3-glucanase are used to increase the effectiveness of lytic peptides by facilitating access of the lytic peptide(s) to the cell membrane of the pathogen. The present invention further relates to transgenic plants that are able to produce lytic peptides and hydrolytic enzymes as well as to methods for controlling said plant pathogens.

It is known that some lytic peptides are active against a broad range of organisms while others produce little or no effect. It is also known that one target of the lytic peptides is the membrane. The amino acid sequence of several lytic peptides with antimicrobial activity are disclosed in WO 89/04371. Hydrolytic enzymes, such as chitinase and β-1,3-glucanase, are known to inhibit fungal growth (Schlumbaum et al., 1986; Mauch et al., 1988; and Burri, 1989). It is also well known that chitinase and β-1,3-glucanase are strongly induced in beans by ethylene treatment. Furthermore, those enzymes can be purified quite easily (Boiler et al., 1983). Lehrer et al. (1986) disclose six antimicrobial peptides (AMPs) from rabbit granulocytes that are structurally homologous to human neutrophil defensins. Three of the rabbit AMPs (NP-1, NP-2 and NP-3a) are disclosed to be effective against *Candida albicans*. Lehrer et al. (1986) further disclose that NP-5, although not directly fungicidal against *C. albicans*, demonstrated what is termed a synergistic effect, an increase in the fungicidal activity of NP-1, when the fungus was exposed to both NP-5 and NP-1. Ogasawara et al. (1973) disclose that the combination of β-1,3-glucanase and chitinase is an effective fungicide and bactericide useful in the treatment of rice. WO 88/00976 discloses the inhibition of crop damage from *Lepidoptera* larvae by expressing genes which code for chitinase enzyme in the target crop.

It is one object of the present invention to provide novel compositions for controlling plant pathogens (preferably fungi, bacteria and nematodes). These novel compositions demonstrate excellent activity against a broad range of pathogens affecting plants.

It is another object of the present invention to provide compositions in which the pathogen's membrane is more easily degraded by lytic peptides.

It is another object of the present invention to provide anti-pathogenically effective agents in which the anti-pathogenically effective activity is inducible.

It is another object of the present invention to provide anti-pathogenically effective compositions employing the above novel combinations of anti-pathogenic agents.

It is a further object of the present invention to provide transgenic plants with anti-pathogenic effective activity based on the expression of one or more lytic peptides which is not lysozyme and one or more chitinases; and/or one or more β-1,3-glucanases in a synergis tically effective amount in the plant cells.

It is another object of the present invention to provide transgenic plants that contain DNA sequences coding for one or more lytic peptides which is not lysozyme in combination with (a) one or more chitinases; and/or (b) one or more β-1,3-glucanases in a synergistically effective amount. It is one feature of the present invention to control plant pathogens by exposing them to one or more chitinases and/or one or more β-1,3-glucanases which degrade the cell wall and make the pathogen's membrane more accessible to lytic peptides, which is not lysozyme.

It is another feature of the present invention that inducible chitinase and β-1,3-glucanase are used in combination with lytic peptides, which is not lysozyme.

It is another feature of the present invention that the combination on one or more chitinases and/or one or more β-1,3-glucanases is effective with a broad range of lytic peptides, which is not lysozyme.

It is one advantage of the present invention that very small amounts of chitinase and β-1,3-glucanase are effective to synergistically increase the inhibitory effects of lytic peptides, which is not lysozyme.

According to the present invention anti-pathogenically effective compositions are provided employing as the active ingredient combinations of one or more lytic peptides, which is not lysozyme and one or more chitinases and/or one or more β-1,3-glacanases in a synergistically effective amount. Lytic peptides attack the pathogen's cell membrane. Because the pathogen's cell membrane is protected by the cell wall, applicants proposed that the use of cell wall lytic enzymes might allow lytic peptides better access to the pathogen's cell membrane. The applicants further hypothesized that a combination of cell wall lytic enzymes with cell membrane lytic peptides would increase the inhibitory effect of the lytic peptides on pathogens. Applicants have found that only very small amounts of hydrolytic enzymes are necessary to synergistically increase the inhibitory effect of all lytic peptides tested.

The term "active ingredient" is used to refer to the combination of one or more lytic peptides which is not lysozyme and one or more chitinases and/or one or more β-1,3-glucanases in a synergistically effective amount which combination functions to inhibit growth of pathogens. The active ingredients of the present invention may be used as one component in anti-pathogenically effective compositions, formulations or preparations.

The term "agriculturally acceptable carriers" is used to refer to those substances, or combinations of substances, that are known in the art of agricultural compositions, and may include, where appropriate, solid or liquid adjuvants, solvents, as well as surfactants and other compounds normally used in agricultural formulations.

As used in the present application, a "pathogenically effective amount" of a substance or composition means an amount which will kill or inhibit the growth of the target plant pathogen when applied to a plant, or when expressed in a transgenic plant.

The term "plant pathogen" includes fungi, bacteria, nematodes and insects. The preferred target pathogens according to the present invention are fungi and bacteria.

The terms "lytic peptide" and "cell membrane lytic peptides" are used to refer to any natural or synthetic peptide or functional derivative thereof which is not lysozyme and which is capable of anti-pathogenically effective activity due to its ability to penetrate, lyse or otherwise impair the pathogen's cell membrane. There are many examples of lytic peptides from animal, plant, insect and microbial sources that may be used in the present invention including but not limited to the mammalian defensins, cecropins, thionins, mellitins, insect defensins, magainins, attacins, dipterins, sapecins, caerulins, xenopsins, or hybrids thereof. For examples of the amino acid sequences of such lytic peptides, see WO 89/11291; WO 86/04356; WO 88/05826; US 4,810,777; and WO 89/04371; Bohlmann et al. (1988); Selsted and Harwig (1987).

Also useful as the lytic peptides of the present invention are synthetic peptides, which may be functional derivatives of one of the lytic peptides above or functional hybrids thereof. One example of such a synthetic peptide is Synthetic Peptide No. 3, which is a derivative of a magainin and has the amino acid sequence SEQ ID NO:1 (Terry et al., 1988).

As used in the present application, the term lytic peptides also includes substances which are active against cell membranes, such as phospholipases. Unlike the above lytic peptides, the phospholipases attack the phospholipids of the cell membrane.

In place of lytic peptides other cell-membrane-degrading components may be employed in the active ingredient of the present invention. For example, it is within the scope of the present invention to employ cell-membrane-degrading detergents such as Triton and SDS.

The terms "hydrolytic enzyme", "cell wall hydrolase," "plant hydrolase" and "cell wall hydrolytic enzyme" are used to refer to chitinase and β-1,3-glucanase.

As used in the present application, the term "constitutive" refers to a hydrolytic enzyme or other substance that is present at all times in a plant cell. The term "inducible" refers to a hydrolytic enzyme or other substance that is present in the plant cell only when the plant is subjected to some stress condition or external stimulus and the production or presence of the hydrolytic enzyme or other substance is thereby activated or increased.

Plant pathogens which may be the targets of the pesticidal compositions of the present invention include members of the following classes: bacteria (for example *Pseudomonas*, *Xanthomonas* and *Erwinia);* fungi, such as Fungi imperfecti (for example, *Botrytis*, *Septoria); Ascomycetes* (for example, *Erysiphe*, *Monilia); Oomycetes* (for example, *Peronospora*, *Phytophthora, Plasmopara, Colletotrichum* and *Pythium); Basidiomycetes* (for example, *Rhizoctonia* and *Puccinia*); as well as to insects and nematodes (for example species of *Meloidogyne*, *Caenorhabditis, Globora, Heterodera* and *Pratylenchus).* For example, the fungal pathogens may include the species *Botrytis cinerea; Colletotrichum lagenarium; Erysiphe graminis*, a wheat pathogen; *Monilia fructicola; Peronospora tabacina; Phytophthora parasitica; Plasmopara viticola; Pythium ultimum*, a soil pathogen; *Rhizoctonia solani*, another soil pathogen; and *Septoria nodorum*, a wheat pathogen.

Target crops to be protected within the scope of the present invention include the following species of plants: maize, cereals (e.g., wheat, barley, rye, oats, rice, sorghum and related crops), beet (e.g., sugar beet and fodder beet), drupes, pomes and soft fruit (e.g., apples, pears, plums, peaches, almonds, cherries, strawberries, raspberries and blackberries), leguminous plants (e.g., beans, lentils, peas, soybeans), oil plants (e.g., rape, mustard, poppy, olives, sunflowers, coconuts, caster oil plants, cocoa beans, groundnuts), cucumber plants (e.g., cucumber, marrows, melons), fibre plants (e.g., cotton, flax, hemp, jute), citrus fruit (e.g., oranges, lemons, grapefruit, mandarins), vegetables (e.g., spinach, lettuce, asparagus, cabbages, carrots, onions, tomatoes, potatoes, paprika), *Lauraceae* (e.g., avocados, cinnamon, camphor), or plants such as tobacco, nuts, coffee, sugar cane, tea, vines, hops, bananas and natural rubber plants, as well as ornamentals.

The relative concentrations of the hydrolytic enzymes in the active ingredient of the present invention is defined in terms of units of activity. The standard unit of concentration ("standard concentration" or "c") of hydrolytic enzyme is defined as the concentration of that enzyme that will produce an absolute activity value equal to that found for the purified hydrolytic enzyme. For example, the standard unit of concentration for chitinase is defined as the concentration of chitinase which will produce an absolute activity of 88.86 nkatal/ml extract. The standard unit of concentration of β-1,3-glucanase is defined as that concentration which demonstrates an absolute activity value equal to that found for the partially purified enzyme, e.g., 76.00 nkatal/ml extract.

The present invention relates to anti-pathogenically effective compositions comprising combinations of one or more lytic peptides which is not lysozyme with one or more chitinases and/or one or more β-1,3-glucanases. In a preferred embodiment of the present invention, both chitinase and β-1,3-glucanase are present in the active ingredient in small amounts, for example, in relative concentrations of about 1/10 of the standard concentration (c), or, more preferably, about 1/100 c. The hydrolytic enzymes most likely allow for greater accessibility to the pathogen's cell membrane for lytic peptides.

In a preferred embodiment, the lytic peptides of the present invention are present in the active ingredient in a concentration from about 0.1 ppm to about 1000 ppm, preferably from about 0.5 ppm to about 500 ppm, especially from about 1 ppm to about 200 ppm.

The concentrations of chitinase and β-1,3-glucanase that were found to be active against the test pathogens, when applied in combination with lytic peptides according to the invention, are present in many plant species, even without a previous stimulus that leads to an induction of these hydrolytic enzymes. However, the naturally present chitinases and β-1,3-glucanases are not pathogenesis-related, and are found in the vacuole rather than in the extracellular space of the plant cell (Boller et al., 1983; Burri, 1989). Thus, in one preferred embodiment of the present invention, the lytic peptide is used in combination with activated hydrolytic enzymes that are already present in a plant. Chitinase and β-1,3-glucanase are both inducible in plants. Thus, in another preferred embodiment of the present invention, the production of chitinase and β-1,3-glucanase is induced using an external stimulus.

Induction may be by chemical means, for example, chemicals known to act as inducers of pathogen related proteins in plants include ethylene, benzoic acid, salicylic acid, polyacrylic acid and substituted derivatives thereof. Induction may also be by other physiological and physical means, such as by high or low temperatures, physical wounding or by any other known inductive means.

The present invention further embraces the preparation of anti-pathogenically effective compositions in which the active ingredient is a combination of one or more lytic peptides which is not lysozyme and one or more chitinases and/or one or more β-1,3-glucanases in a synergistically effective amount. The active ingredient is homogeneously mixed with one or more compounds or groups of compounds described herein. The present invention also relates to methods of protecting plants from the attack of plant pathogens, which comprise application of the active ingredient, or anti-pathogenically effective compositions containing the active ingredient in a synergistically effective amount to the plant or to the pathogen's habitat.

The active ingredients of the present invention are normally applied prophylacticly or curatively in the form of compositions together with one or more agriculturally acceptable carriers, and can be applied to the crop area or plant to be treated, simultaneously or in succession, with further compounds. These compounds can be both fertilizers or micronutrient donors or other preparations that influence plant growth. They can also be selective herbicides, insecticides, fungicides, bactericides, nematicides, molluscicides or mixtures of several of these preparations, if desired together with further carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

A preferred method of applying active ingredients of the present invention or an agrochemical composition which contains at least one of the active ingredients is leaf application. The number of applications and the rate of application depend on the intensity of infestation by the corresponding pathogen. However, the active ingredients can also penetrate the plant through the roots via the soil (systemic action) by impregnating the locus of the plant with a liquid composition, or by applying the compounds in solid form to the soil, e.g. in granular form (soil application). The active ingredients may also be applied to seeds (coating) by impregnating the seeds either with a liquid formulation containing active ingredients, or coating them with a solid formulation. In special cases, further types of application are also possible, for example, selective treatment of the plant stems or buds.

In order for the anti-pathogenically effective compositions to inhibit growth of a pathogen such as the fungus *S. nodorum*, it is assumed that the pathogen first comes into contact with the hydrolytic enzymes according to the invention. After contact with chitinase and β-1,3-glucanase, the fungal membrane is more accessible to lytic peptides according to the invention. Additionally, it is important to note that a plant's response in defense of pathogenic infection consists of many different mechanisms. While the combinations of chitinase, β-1,3-glucanase and various lytic peptides, which is not lysozyme, show a considerable inhibition of pathogenic growth by destroying the pathogen's cell wall and the pathogen's membrane, they most likely assist a plant's anti-pathogenic ability by providing access to other anti-pathogenic substances which are produced during a plant's hypersensitive response. Thus, the combinations of one of more chitinases, and/or one or more β-1,3-glucanases and one or more lytic peptides, which is not lysozyme, in a synergistically effective amount of the present invention are effective agents for the control of pathogens.

The active ingredients are used in unmodified form or, preferably, together with the adjuvants conventionally employed in the art of formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objectives and the prevailing circumstances. Advantageous rates of application are normally from 50 g to 5 kg of active ingredient (a.i.) per hectare (ha), preferably from 100 g to 2 kg a.i./ha, most preferably from 200 g to 500 g a.i./ha.

The formulations, compositions or preparations containing the active ingredients and, where appropriate, a solid or liquid adjuvant, are prepared in known manner, for example by homogeneously mixing and/or grinding the active ingredients with extenders, for example solvents, solid carriers and, where appropriate, surface-active compounds (surfactants).

Suitable solvents include aromatic hydrocarbons, preferably the fractions having 8 to 12 carbon atoms, for example, xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol monomethyl or monoethyl ether, ketones such as cyclohexanone, strongly polar solvents such as N-methyl-2-pyrrolidone, dimethyl sulfoxide or dimethyl formamide, as well as epoxidized vegetable oils such as epoxidized coconut oil or soybean oil; or water.

The solid carriers used e.g. for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredient to be used in the formulation, suitable surface-active compounds are nonionic, cationic and/or anionic surfactants having good emulsifying, dispersing and wetting properties. The term "surfactants" will also be understood as comprising mixtures of surfactants.

Suitable anionic surfactants can be both water-soluble soaps and water-soluble synthetic surface-active compounds.

Suitable soaps are the alkali metal salts, alkaline earth metal salts or unsubstituted or substituted ammonium salts of higher fatty acids (chains of 10 to 22 carbon atoms), for example the sodium or potassium salts of oleic or stearic acid, or of natural fatty acid mixtures which can be obtained for example from coconut oil or tallow oil. The fatty acid methyltaurin salts may also be used.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, alkaline earth metal salts or unsubstituted or substituted ammoniums salts and have a 8 to 22 carbon alkyl radical which also includes the alkyl moiety of alkyl radicals, for example, the sodium or calcium salt of lignosulfonic acid, of dodecylsulfate or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethanolamine salts of dodecylbenzenesulfonic acid, dibutylnapthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide.

Non-ionic surfactants are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediamine propylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxyethoxyethanol. Fatty acid esters of polyoxyethylene sorbitan and polyoxyethylene sorbitan trioleate are also suitable non-ionic surfactants.

Cationic surfactants are preferably quaternary ammonium salts which have, as N-substituent, at least one C₈-C₂₂alkyl radical and, as further substituents, lower unsubstituted or halogenated alkyl, benzyl or lower hydroxyalkyl radicals. The salts are preferably in the form of halides, methylsulfates or ethylsulfates, e.g. stearyltrimethylammonium chloride or benzyldi(2-chloroethyl)ethylammonium bromide.

The surfactants customarily employed in the art of formulation are described, for example, in McCutcheon's Detergents and Emulsifiers Annual (1979), and Sisely and Wood (1980).

The agrochemical compositions usually contain from about 0.1 to about 99 %, preferably about 0.1 to about 95 %, and most preferably from about 3 to about 90 % of the active ingredient, from about 1 to about 99.9 %, preferably from about 1 to about 99 %, and most preferably from about 5 to about 95 % of a solid or liquid adjuvant, and from about 0 to about 25 %, preferably about 0.1 to about 25 %, and most preferably from about 0.1 to about 20 % of a surfactant.

Whereas commercial products are preferably formulated as concentrates, the end user will normally employ dilute formulations.

The present invention also relates to transgenic plants that contain DNA sequences coding for at least one lytic peptide and at least one chitinase and/or β-1,3-glucanase. Transgenic plants containing DNA sequences coding for chitinase and β-1,3-glucanase are disclosed in EP 392 225 and EP 353 191.

The present invention also relates to a method of preparing a transgenic plant which is able to synthesize lytic enzymes, and crossing said plants using conventional breeding techniques.

Preferably the method comprises preparing a first homozygous transgenic plant comprising a recombinant DNA sequence encoding a cell membrane-degrading component, preparing a second homozygous transgenic plant comprising a recombinant DNA sequence encoding β-1,3-glucanase, preparing a third homozygous transgenic plant comprising a recombinant DNA sequence encoding chitinase, and crossing the three homozygous plants using conventional breeding techniques.

In transgenic plants, the synthesis of lytic peptides and hydrolytic enzymes may be induced by use of an inducible expression system comprising an inducible gene and an inducing regulator. For example, more than ten of the tobacco PR (pathogen related) protein genes are chemically inducible (van Loon, 1985; Jamet and Fritig, 1986). Three of these genes, PR-2, PR-N and PR-O have been shown to have β-1,3-glucanase activity (Kauffman et al., 1987), and two, PR-P and PR-Q, have been shown to have chitinase activity (Legrand et al., 1987). Thus, the present invention includes transgenic plants which express a gene encoding at least one lytic peptide followed by treatment with an inducing regulator which induces the accumulation of at least one natural or foreign hydrolytic enzyme.

The invention is illustrated in more detail by the following examples, without implying any restriction to what is described therein.

### Deposits with ATCC

The following deposits have been made with the ATCC in accordance with the Budapest Treaty:
ATCC 40426, deposited February 12, 1988
ATCC 40586, deposited March 22, 1989

### Examples

### Example 1: Purification of chitinase and β-1,3-glucanase from ethylene-treated bean leaves

Step 1: Ethylene treatment and plant material. Seeds of *Phaseolus vulgaris L.* cv. *saxa* are grown in vermiculite under a 16 hr light:8 hr dark regime at 20°C. Twelve day old bean plants are incubated in air-tight plastic chambers with a volume of 0.2 m³. Control plants are incubated in identical chambers in ethylene-free air. After 48 hours, the primary leaves are harvested and frozen at -20°C.

Step 2: Crude enzyme preparation. 659.7 g frozen leaf tissue is homogenized in 0.1 M sodium citrate buffer at pH 5.0 (2 ml/g fresh weight) with a Turmix homogenizer at top speed. The homogenate is centrifuged (25 min, 11000 x g) and 100 ml of the supernatant are used as a crude enzyme preparation. The proteins in the remaining crude extract are precipitated by adding ammonium sulfate to 95 % saturation. After centrifugation (30 min, 11000 x g) the sediment is redissolved in 100 mM Tris/HCl pH 8.0 and dialyzed against 5 mM Tris/HCl pH 8.0.

Step 3: Enzyme purification. The dialyzed crude protein fraction is passed through a diethylaminoethyl (DEAE)-trisacryl (IBF, Clichy, F) column equilibrated in 5 mM Tris/HCl pH 8.0. Buffer containing the basic proteins which pass through the column unretarded is brought to a final concentration of 20 mM NaHCO₃. The pH is raised to 8.4 with 1 M NaOH and the liquid is loaded onto a column of regenerated chitin (Molano et al., 1977) equilibrated in 20 mM NaHCO₃. The flow-through is collected and the pH is brought to 5.5 with 1 M HCI. The proteins are dialyzed against water and treated three times with a small amount of regenerated chitin, followed by centrifugation to remove traces of chitinase. The fractions containing a high activity of β-1,3-glucanase are then lyophilized. They are the source of partially purified β-1,3-glucanase.

The chitin column is washed with 20 mM NaHCO₃, followed by 20 mM sodium acetate buffer pH 5.5. Chitinase is eluted with 100 mM acetic acid in an elution volume of 31 ml. The pH of the eluate is brought to 5.5 with 1 M KOH and the protein is dialyzed against water and lyophilized. This protein will be the source of purified chitinase.

Step 4: Determination of protein. Protein is measured as described by Bradford (1976).

Step 5a: Chitinase assay. Chitinase activity is measured as described by Molano et al. (1977).

Step 5b: β-1,3-glucanase assay. β-1,3-glucanase activity is measured as described by Dygert et al. (1965).

Step 6: Enzyme induction in ethylene-treated bean leaves. A 48 hr ethylene treatment leads to a strong induction of chitinase and β-1,3-glucanase, as demonstrated by the results in Table 1:

**Table 1:**

| Chitinase and β-1,3-glucanase activity in ethylene-treated bean leaves | |
|---|---|
| Specific chitinase activity (nkatal/mg protein) | 45.95 |
| Absolute chitinase activity (nkatal/ml extract) | 54.71 |
| Specific β-1,3-glucanase activity (nkatal/mg protein) | 28.72 |
| Absolute β-1,3-glucanase activity (nkatal/ml extract) | 34.21 |

Step 7: Enzyme purification. Table 2 shows the purification protocol of chitinase and β-1,3-glucanase. Chitinase is 33 times purified with a yield of 21.6 %. β-1,3-glucanase is purified with a purification factor of 3.0 and a yield of 33.5 %. SDS polyacrylamide gel electrophoresis shows that β-1,3-glucanase is quite highly purified after these purification steps (Burri, 1989).

Step 8: Induction of chitinase and β-1,3-glucanase in bean leaves. In the bean leaves used for this experiment, the enzymes chitinase and β-1,3-glucanase are slightly less induced than expected. For example, in Burri (1989), the average of five ethylene treatments gave an absolute chitinase activity of 88.86 + 6.65 nkatal/ml extract and an absolute β-1,3-glucanase activity of 76.00 ± 6.77 nkatal/ml extract. Therefore, the unit of standard concentration for chitinase (c= 1) is defined as the concentration of chitinase necessary to attain an activity of 88.86 nkatal/ml extract. The relative concentration of chitinase extracts are defined in terms of fractions of that activity. The relative concentrations of partially purified β-1,3-glucanase are defined in terms of fractions of an activity of 76.00 nkatal/ml extract.

Step 9: Chitinase purification. Chitinase is purified by affinity chromatography. The purification factor is comparable to that in Burri (1989) (32 %).

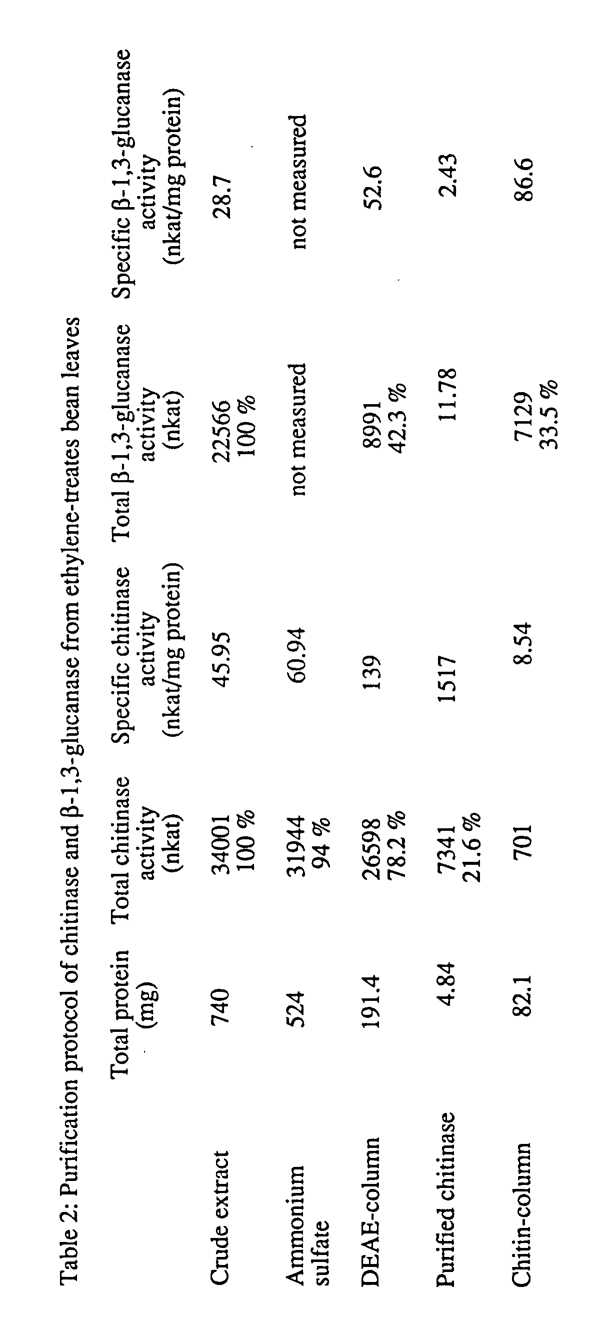

Step 10: β-1,3-glucanase purification. The yield of 35.5 % is approximately one-third higher than that in Burri (1989) at that purification step while the purification factor of 3.0 is not as high as previously observed.

### Example 2: Synthesis of lytic peptides

Lytic peptides are synthesized using an Applied Biosystems Model 430 Peptide Synthesizer according to the manufacturer's recommendations. Peptides are purified by HPLC.

### Example 3: The inhibitory effect of lytic peptides and hydrolytic enzymes against S. nodorum

Microtiter plates are selected to investigate the inhibitory effect of lytic peptides and hydrolytic enzymes against *S. nodorum*. Test substances are added to 90 µl liquid pea medium which has been previously inoculated with 2.0 x 10⁴ spores/ml medium of the test pathogen to give a final volume of 100 µl. Table 3 illustrates the composition of test solutions which are tested in duplicate/triplicate samples. The blank value of the test substances are determined by adding them to uninoculated liquid pea medium. The plates are evaluated after incubation in a wet chamber at 23°C on a shaker in the dark for 48 hours.

The growth of the pathogen is recorded by measuring the absorbance of the medium at 595 nm. The average of duplicate/triplicate samples is calculated and the corresponding enzyme blank is subtracted to give the net absorption of the growing mycelium. The inhibitory effect of the test substances used is determined by calculating the growth of the pathogen as a percentage of water control.

The results of the above described test are compiled in Table 4. When applied as single test solutions, all test substances are able to inhibit growth of *S. nodorum* to a limited extent, depending upon the concentrations used. The combination of partially purified β-1,3-glucanase and lytic peptide is found to inhibit pathogenic growth more strongly than combinations of purified chitinase and lytic peptide. This is consistent with the finding that *S. nodorum* is more sensitive to partially purified β-1,3-glucanase than to purified chitinase. As Table 4 demonstrates, additions of 1/100 c of either enzyme to a particular lytic peptide concentration only slightly increases the inhibitory effect of the test substance in comparison with the lytic peptide alone. Combinations of 1/10 c of either enzyme with a particular lytic peptide concentration more strongly inhibit the test pathogen than combinations of 1/100 c enzyme with lytic peptide. It is especially interesting that combinations of both enzymes, either in 1/10 c or 1/100 c concentration, with lytic peptides strongly increase the inhibitory effect of the test substances.

**Table 3:**

| Test solutions | | | |
|---|---|---|---|
| Sample | Purified Chitinase | Purified β-1,3-Glucanase | Lytic Peptide |
| | (c) | (c) | (ppm) |
| 0 | - | - | - |
| 1 | 1/10 | - | - |
| 2 | 1/100 | - | - |
| 3 | - | 1/10 | - |
| 4 | - | 1/100 | - |
| 5 | - | - | 20 |
| 6 | - | - | 60 |
| 7 | - | - | 200 |
| 8 | 1/10 | 1/10 | - |
| 9 | 1/10 | 1/100 | - |
| 10 | 1/100 | 1/10 | - |
| 11 | 1/100 | 1/100 | - |
| 12 | 1/10 | - | 20 |
| 13 | 1/10 | - | 60 |
| 14 | 1/10 | - | 200 |
| 15 | 1/100 | - | 20 |
| 16 | 1/100 | - | 60 |
| 17 | 1/100 | - | 200 |
| 18 | - | 1/10 | 20 |
| 19 | - | 1/10 | 60 |
| 20 | - | 1/10 | 200 |
| 21 | - | 1/100 | 20 |
| 22 | - | 1/100 | 60 |
| 23 | - | 1/100 | 200 |
| 24 | 1/10 | 1/10 | 20 |
| 25 | 1/10 | 1/10 | 60 |
| 26 | 1/10 | 1/10 | 200 |
| 27 | 1/100 | 1/100 | 20 |
| 28 | 1/100 | 1/100 | 60 |
| 29 | 1/100 | 1/100 | 200 |

Water control: 10 µl distilled water are added to 90 µl spore solution.

**Table 4:**

| Results of test solutions, fungal growth in % of control | | | | |
|---|---|---|---|---|
| Test solution | Synthetic peptide no. 3 | Barley thionin β | Mellitin | Enzymes |
| 0 (Control) | - | - | - | 100.00 |
| 1 | - | - | - | 78.60 |
| 2 | - | - | - | 93.68 |
| 3 | - | - | - | 50.18 |
| 4 | - | - | - | 81.75 |
| 5 | 80.35 | 86.32 | 79.65 | - |
| 6 | 73.51 | 63.86 | 65.96 | - |
| 7 | 65.96 | 60.70 | 27.37 | - |
| 8 | - | - | - | 34.21 |
| 9 | - | - | - | 45.61 |
| 10 | - | - | - | 39.82 |
| 11 | - | - | - | 60.88 |
| 12 | 68.42 | 62.81 | 62.81 | - |
| 13 | 63.51 | 49.47 | 57.54 | - |
| 14 | 61.75 | 47.02 | 22.46 | - |
| 15 | 77.89 | 75.44 | 73.33 | - |
| 16 | 71.58 | 62.81 | 65.26 | - |
| 17 | 64.21 | 57.19 | 25.61 | - |
| 18 | 46.67 | 13.68 | 14.74 | - |
| 19 | 30.88 | 12.98 | 11.93 | - |
| 20 | 27.89 | 5.26 | 6.32 | - |
| 21 | 65.26 | 54.74 | 61.40 | - |
| 22 | 63.51 | 46.67 | 57.19 | - |
| 23 | 53.68 | 39.30 | 25.96 | - |
| 24 | 9.47 | 7.72 | 10.53 | - |
| 25 | 8.42 | 2.22 | 7.37 | - |
| 26 | 3.86 | 0.00 | 0.00 | - |
| 27 | 40.7 | 42.46 | 22.72 | - |
| 28 | 36.49 | 32.98 | 22.11 | - |
| 29 | 31.23 | 28.77 | 18.75 | - |

Hydrolytic enzymes are purified from leaves by the procedure described in Example 1. Lytic peptides are tested at 20 ppm, 60 ppm and 200 ppm concentrations.

### Example 4: Formulations of anti-pathogenically effective compositions employing liquid compositions of lytic peptides and hydrolytic enzymes as the active ingredient.

In the following, percentages of compositions are given by weight.

| 1. Emulsifiable concentrates: | a | b | c |
|---|---|---|---|
| Active ingredient | 20% | 40% | 50% |
| Calcium dodecylbenzenesulfonate | 5 % | 8 % | 6 % |
| Castor oil polyethlene glycol ether (36 mol ethylene oxide) | 5 % | - | - |
| Tributylphenol polyethylene glycol ether (30 mol ethylene oxide) | - | 12 % | 4 % |
| Cyclohexanone | - | 15 % | 20 % |
| Xylene mixture | 70 % | 25 % | 20 % |

Emulsions of any required concentration can be produced from such concentrates by dilution with water.

| 2. Solutions: | a | b | c | d |
|---|---|---|---|---|
| Active ingredient | 80 % | 10 % | 5 % | 95 % |
| Ethylene glycol monomethyl ether | 20 % - | - | - | - |
| Polyethylene glycol 400 | - | 70 % - - - | | |
| N-methyl-2-pyrrolidone | - | 20 % - | - | - |
| Epoxidised coconut oil | - | - | 1 % | 5 % |
| Petroleum distillate (boiling range 160°C to 190°C) | - | - | 94 % | - |

These solutions are suitable for application in the form of microdrops.

| 3. Granulates: | a | b |
|---|---|---|
| Active ingredient | 5 % | 10 % |
| Kaolin | 94 % | - |
| Highly dispersed silicic acid | 1 % | - |
| Attapulgite | - | 90 % |

The active ingredient is dissolved in methylene chloride, the solution is sprayed onto the carrier, and the solvent is subsequently evaporated off in vacuo.

| 4. Dusts: | a | b |
|---|---|---|
| Active ingredient | 2 % | 5 % |
| Highly dispersed silicic acid | 1 % | 5 % |
| Talcum | 97 % | - |
| Kaolin | - | 90 % |

Ready-to-use dusts are obtained by intimately mixing the carriers with the active ingredient.

### Example 5: Formulation of anti-pathogenically effective compositions employing solid compositions of lytic peptides and hydrolytic enzymes as the active ingredient

In the following, percentages of compositions are by weight.

| 1. Wettable powders: | a | b | c |
|---|---|---|---|
| Active ingredient | 20 % | 60 % | 75 % |
| Sodium lignosulfonate | 5 % | 5 % | - |
| Sodium lauryl sulfate | 3 % - | | 5 % |
| Sodium diisobutylnaphthalene-sulfonate | - | 6 % | 10 % |
| Octylphenol polyethylene glycol ether (7 to 8 moles of ethylene oxide) | - | 2 % | - |
| Highly dispersed silicic acid | 5 % | 27 % | 10 % |
| Kaolin | 67 % - | - | - |

The active ingredient is thoroughly mixed with the adjuvants and the mixture is thoroughly ground in a suitable mill, affording wettable powders which can be diluted with water to give suspensions of the desired concentrations.

| 2. Emulsifiable concentrate: | |
|---|---|
| Active ingredient | 10 % |
| Octylphenol polyethylene glycol ether (4 to 5 moles of ethylene oxide) | 3 % |
| Calcium dodecylbenzenesulfonate | 3 % |
| Castor oil polyglycol ether (36 moles of ethylene oxide) | 4 % |
| Cyclohexanone | 30 % |
| Xylene mixture | 50 % |

Emulsions of any required concentration can be obtained from this concentrate by dilution with water.

| 3. Dusts: | a | b |
|---|---|---|
| Active ingredient | 5 % | 8 % |
| Talcum | 95 % | - |
| Kaolin | - | 92 % |

Ready-to-use dusts are obtained by mixing the active ingredient with the carriers, and grinding the mixture in a suitable mill.

| 4. Extruder granulate: | |
|---|---|
| Active ingredient | 10 % |
| Sodium lignosulfonate | 2 % |
| Carboxymethylcellulose | 1 % |
| Kaolin | 87 % |

The active ingredient is mixed and ground with the adjuvants, and the mixture is subsequently moistened with water. The mixture is extruded and then dried in a stream of air.

| 5. Coated granulate: | |
|---|---|
| Active ingredient | 3 % |
| Polyethylene glycol 200 | 3 % |
| Kaolin | 94 % |

The finely ground active ingredient is uniformly applied, in a mixer, to the kaolin moistened with polyethylene glycol. Non-dusty coated granulates are obtained in this manner.

| 6. Suspension concentrate: | |
|---|---|
| Active ingredient | 40 % |
| Ethylene glycol | 10 % |
| Nonylphenol polyethylene glycol | 6 % |
| (15 moles of ethylene oxide) | |
| Sodium lignosulfonate | 10 % |
| Carboxymethylcellulose | 1 % |
| 37 % aqueous formaldehyde solution | 0.2 % |
| Silicone oil in 75 % aqueous emulsion | 0.8 % |
| Water | 32 % |

The finely ground active ingredient is intimately mixed with the adjuvants, giving a suspension concentrate from which suspensions of any desired concentration can be obtained by dilution with water.

### Example 6: Construction of binary vectors pCGN1781C and pCGN1782C

pCIB1005B, which contains a double 35S cauliflower mosaic virus (CaMV) promoter operably linked to a gene encoding the expression of basic glucanase is constructed as follows:

The plasmid pGLN17 is a hybrid cDNA encoding the basic β-1,3-glucanase from *N. tabacum* (Shinshi et al., 1988) constructed by fusing the 5' end of the pGL31 clone and the 3' end of the pGL36 clone. This hybrid cDNA has the sequence SEQ ID NO:9.

The cDNA is truncated at the 5' end and does not encode the entire signal peptide. In order to make transgenic plants in which this protein is properly targeted (i.e., the central vacuole), it is necessary to add this sequence back on to the truncated cDNA. Therefore, the double CaMV 35S expression cassette is constructed in a two step process. In the first step the signal peptide of the cDNA is replaced by a signal peptide encoded in the genomic clone. In the second step, this "repaired cDNA" is moved into the expression vector.

The plasmid pSGL2 is a subclone of the pGLN17 cDNA. This plasmid is digested with ClaI and EcoRI and the 1 kb fragment containing the glucanase cDNA is isolated from a LGT agarose gel. The pBluescript plasmid is digested with EcoRI, treated with CLAP and purified on a LGT agarose gel.

The plasmid pBS-Gluc 39.1 contains a 4.4 kb insert which includes the glucanase coding sequence, about 1.5 kb of 5' flanking sequence, a 600 bp intron, and about 1 kb of 3' flanking sequence. This plasmid is used as a template in a PCR experiment containing two primers with the sequences SEQ ID NO:10 or SEQ ID NO:11.

The result of this amplification is to produce a fragment to replace the truncated 5' end of the glucanase cDNA. A single-base mutation creating an EcoRI site is introduced to facilitate cloning experiments. The PCR product is digested with EcoRI and ClaI and fragments are separated on a 2.0 % LGT agarose gel. A 120 bp band is excised, mixed with the 1 kb ClaI-EcoRI fragment from pSGL2 and the purified, EcoRI digested bluescript vector, ligated and transformed as described above. Transformants are screened for the presence of the insert and one plasmid with the proper structure is designated pCIB1009.

The plasmid pCIB1009 is digested with EcoRI and the 1.2 kb fragment is purified on a LGT agarose gel. The plasmid pCGN1761 is digested with EcoRI, treated with CIAP, purified on a LGT agarose gel, mixed with the 1.2 kb EcoRI fragment, and then ligated and transformed. Transformants are screened for the presence of the insert. One plasmid, in which the glucanase cDNA is in a sense orientation relative to the CaMV promoter is designated as pCIB1005B.

The 4.9 kb XbaI fragment of pCIB1005B is subcloned into XbaI digested pCGN1540 in such an orientation that the double 35S promoter fragment is proximal to the plant selectable marker gene of pCGN 1540 to create pCGN 1781C.

The construction of plasmid pCIB1007, which contains a double 35S CaMV promoter operably linked to a gene encoding the expression of basic chitinase is as follows:

The plasmid pSCH10 contains a cDNA insert of the tobacco basic chitinase which is similar to the insert in pCHN50 (Shinshi et al., 1987) but with an extension of 81 bp on the 5' end (SEQ ID NO:12).

pSCH10 is digested with BamHI and then ligated with a molecular adaptor (SEQ ID NO:13) as described in Example 5. The ligation product is then purified and digested with EcoRI and the 1.2 kb fragment containing the adapted chitinase cDNA is purified from a LGT agarose gel.

This fragment is mixed with EcoRI digested, CIAP treated pCGN1761 which is also purified from a LGT agarose gel and the mixture is ligated and transformed. Transformants are screened for the chitinase cDNA insert and one plasmid which contains the chitinase cDNA in a sense orientation relative to the CaMV promoter is designated as pCIB1007.

The 4.8 kb XbaI fragment of pCGN1007 is cloned into XbaI digested pCGN1540 in such an orientation that the double 35S promoter fragment is proximal to the plant selectable marker gene of pCGN1540 to create the binary vector pCGN1782C.

### Example 7: Construction of a gene encoding synthetic lytic peptide No. 3

The synthetic lytic peptide No. 3 as described in the above examples has the amino acid sequence SEQ ID NO: 1.

A synthetic gene encoding this peptide is constructed in several steps. First a gene encoding the mature (processed) protein is synthesized and cloned, then a signal peptide and 5' leader sequence for expression in plants and secretion are added, finally a 3' untranslated sequence and 3' mRNA processing site are added. This results in a gene encoding synthetic peptide No. 3 which can be transformed into a plant and which will direct the synthesis of a lytic peptide which will be secreted extracellularly.

### A. Construction of the coding sequence for the mature peptide

Two oligonucleotides are synthesized, Oligo 1 and Oligo 2, with the sequences SEQ ID NO:2 and SEQ ID NO:3 are synthesized using β-cyanoethyl-phosphoramidite chemistry on an Applied Biosystems 380A synthesizer and are purified using OPC-based purification (Applied Biosystems) as described by the supplier. The purified oligonucleotides are first kinased with polynucleotide kinase and then annealed for one hour at 65°C. The reaction is cooled to 37°C and T4 DNA ligase added along with extra ATP and the ligation is allowed to incubate a further hour. The reaction is then heated to 65°C for 1 min to inactivate the ligase and then diluted and made 1 X in the recommended EcoRI buffer. The ligation reaction is then digested with EcoRI. The DNA is phenol extracted and precipitated with 0.3 M sodium acetate and two volumes of ethanol. The precipitate is collected by centrifugation and resuspended in 10 mM TE buffer (Sambrook et al., 1989). The DNA is electrophoresed on a 3.0 % low gelling temperature agarose gel and the band containing the synthetic gene is excised and ligated into the EcoRI site of pBluescript (Stratagene). The ligation reaction is transformed into *E*. *coli* and colonies are selected and putative positive constructs are analyzed first by restriction digestion and then by DNA sequencing. One construct which is designated pBSlypep3 is determined to contain the correct sequence and is chosen for further work.

### B. Addition of a 5' flanking sequence and signal peptide to pBSlypep3

The 5' leader and signal peptide of the PR-la gene are added to the syntheic gene using a PCR based in vitro gene fusion technique as described by Ho et al. (1989). In this technique a gene fusion is made in vitro by creating two fragments with overlapping ends by PCR. In a subsequent reaction these two fragments are then fused, again by PCR, to generate a perfect fusion between the two molecules. This strategy is used to fuse the PR-la signal peptide and leader to the coding sequence for the lytic peptide. To accomplish this fusion four oligonucleotides are synthesized and purified as described above: SEQ ID NO:4, SEQ ID NO:5, SEQ ID NO:6 and SEQ ID NO:7.

The oligos 3 (SEQ ID NO:4) and 4 (SEQ ID NO:5) are complementary to each other and contain the DNA sequence desired for the fusion between the PR-la signal and leader and the lytic peptide coding sequence. The desired fusion is diagrammed below (SEQ ID NO:8).

The oligo 5 (SEQ ID NO:6) has the same sequence as the 5' end of the PR-la cDNA and it contains on the 5' end a sequence encoding an EcoRI restriction site.

The oligo 6 (SEQ ID NO:7) is complementary to the 3' end of the synthetic gene encoding lyric peptide 3 and also contains an EcoRI site at the 5' end.

In order to fuse the two pieces of DNA, two PCR reactions are set up. One which uses Oligo 3 (SEQ ID NO:4) and Oligo 6 (SEQ ID NO:7) as primers and the plasmid pBSLypep3 as a template and the other which uses Oligo 4 (SEQ ID NO:5) and Oligo 5 (SEQ ID NO:6) as primers and the plasmid pBS-PR1013Cla (ATCC 40426) as a template. The PCR reactions are carried out using the GeneAmp kit (Perkin Elmer/CETUS) as suggested by the suppliers. The PCR products are analyzed by agarose gel electrophoresis. They are then purified and an aliquot of each is used in a second stage PCR reaction. In this reaction the primers, Oligo 5 (SEQ ID NO:6) and Oligo 6 (SEQ ID NO:7), are used to amplify the product. In this way the two pieces of DNA are fused in the predetermined manner explained above. The reaction products are analyzed by agarose gel electrophoresis. The DNA is then phenol extracted and ethanol precipitated and the DNA resuspended and digested with EcoRI. This DNA is then purified by electrophoresis on a low-gelling temperature agarose gel and ligated into pBluescript which is digested with EcoRI and treated with CIAP. The DNA is transformed into *E*. *coli* and transformants are selected. Positive plasmid constructs are screened by restriction digestion and confirmed for the anticipated structure by DNA sequencing. One such construct is designated pBSLP5'PR1 and is used for subsequent experiments.

### C. Subcloning the lytic peptide gene into a double 35S expression vector

The synthetic lytic peptide gene with a 5' leader sequence and a signal peptide is subcloned into the plant expression vector pCGN1761 (a double CaMV 35S promoter/-terminator cassette containing ampicillin resistance).

pCGN1761 contains a double CaMV 35S promoter and the *tm-1* 3' region with an EcoRI site between contained in a pUC-derived plasmid backbone. The promoter-EcoRI-3' processing site cassette is bordered by multiple restriction sites for easy removal. The plasmid is derived by a series of steps (see below) from an initial double-35S plasmid, pCGN2113, which itself is derived from pCGN164, and pCGN638.

pCGN1431 also contains the double CaMV 35S promoter and the *tm-1* 3' region with a multiple cloning site between them. This promoter/terminator cassette is contained in a pUC-derived vector which contains a chloramphenicol rather than ampicillin resistance gene. The cassette is bordered by multiple restriction sites for easy removal.

### a. Construction of pCGN986

pCGN986 contains a 35S CaMV 35 promoter and a T-DNA *tm-1* 3'-region with multiple restriction sites between them. pCGN986 is derived from another plasmid, pCGN206, containing a CaMV 35S promoter and a different 3' region, the CaMV region VI 3'-end. The CaMV 35S promoter is cloned as an AluI fragment (bp 7144-7734) (Gardner et al., 1981) into the HincII site of M13mp7 (Messing et al., 1981) to create C614. An EcoRI digest of C614 produces the EcoRI fragment from C614 containing the 35S promoter which is cloned into the EcoRI site of pUC8 (Vieira and Messing, 1982) to produce pCGN147.

pCGN148a containing a promoter region, selectable marker (Kanamycin with 2 ATGs) and 3' region, is prepared by digesting pCGN528 with BglII and inserting the BamHI-BglII promoter fragment from pCGN147. This fragment is cloned into the BglII site of pCGN528 so that the BglII site is proximal to the kanamycin gene of pCGN528.

The shuttle vector, pCGN528, used for this construct is made as follows: pCGN525 is made by digesting a plasmid containing Tn5 which harbors a kanamycin gene (Jorgensen et al., 1979) with HindIII-BamHI and inserting the HindIII-BamHI fragment containing the kanamycin resistance gene into the HindIII-BamHI sites in the tetracycline gene of pACYC184 (Chang and Cohen, 1978) pCGN526 is made by inserting the BamHI fragment 19 of pTiA6 (Thomashow et al., 1980) modified with XhoI linkers inserted into the SmaI site, into the BamHI site of pCGN525. pCGN528 is obtained by deleting the small XhoI and religating.

pCGN149a is made by cloning the BamHI Kanamycin gene fragment from pMB9KanXXI into the BamHI site of pCGN148a. pMB9KanXXI is a pUC4K variant (Vieira and Messing, 1982) which has the Xhol site missing but contains a function kanamycin gene from Tn903 to allow for efficient selection in *Agrobacterium.*

pCGN149a is digested with HindIII and BamHI and ligated to pUC8 digested with HindIII and BamHI to produce pCGN169. This removes the Tn903 kanamycin marker. pCGN565 and pCGN169 are both digested with HindIII and PstI and ligated to form pCGN203, a plasmid containing the CaMV 35S promoter and part of the 5'-end of the TN5 kanamycin gene (up to the PstI site, Jorgensen et al., 1979). A 3' regulatory region is added to pCGN203 from pCGN204 (an EcoRI fragment of CaMV (bp 408-6105) containing the 3' region of gene VI subcloned into pUC18 (Gardner et al., 1981) by digestion with HindIII and PstI and ligation. The resulting cassette, pCGN206, is the basis for the construction of pCGN986.

The pTiA6 T-DNA *tm-1* 3'-sequences are subcloned from the Bam19 T-DNA fragment (Thomashow et al., 1980) as a BamHI-EcoRI fragment (nucleotides 9062 to 12, 823, numbering as in Barker et al., 1983) and combined with the pACYC184 (Chang and Cohen, 1978) origin of replication as an EcoRI-HindII fragment and a gentamycin resistance marker (from plasmid pLB41), as a BamHI-HindII fragment to produce pCGN417.

The unique SmaI site of pCGN417 (nucleotide 11,207 of the Baml9 fragment) is changed to a SacI site using linkers and the BamHI-SacI fragment is subcloned into pCGN565 to give pCGN971. The BamHI site of pCGN971 is changed to an EcoRI site using linkers to yield pCGN971E. The resulting EcoRI-SacI fragment of pCGN971E, containing the *tm-1* 3' regulatory sequences, is joined to pCGN206 by digestion with EcoRI and SacI to give pCGN975. The small part of the Tn5 kanamycin resistance gene is deleted from the 3'-end of the CaMV 35S promoter by digestion with Sail and BglII, blunting the ends and ligation with Sail linkers. The final expression cassette pCGN986 contains the CaMV 35S promoter followed by two Sail sites, an XbaI site, BamHI, SmaI Kpnl and the *tm-1* 3'region (nucleotides 11207-9023 of the T-DNA).

### b. Construction of pCGN164

The AluI fragment of CaMV (bp 7144-7735) (Gardner et al., 1981) is obtained by digestion with AluI and cloned into the HincII site of M13mp7 (Vieira and Messing, 1982) to create C614. An EcoRI digest of C614 produces the EcoRI fragment from C614 containing the 35S promoter which is cloned into the EcoRI site of pUC8 (Vieira and Messing, 1982) to produce pCGN146. To trim the promoter region, the BglII site (bp7670) is treated with BglII and Bal31 and subsequently a BglII linker is attached to the Bal31 treated DNA to produce pCGN147. PCGN147 is digested with EcoRI HphI and the resultant EcoRI-HphI fragment containing the 35S promoter is ligated into EcoRI-SmalI digested M13mp8 (Vieira and Messing, 1982) to create pCGN164.

### c. Construction of pCGN638

Digestion of CaMV10 (Gardner et al., 1981) with BglII produces a BglII fragment containing a 35S promoter region (bp 6493-7670) which is ligated into the BamHI site of pUC19 (Norrander et al., 1983) to create pCGN638.

### d. Construction of pCGN2113

pCGN164 is digested with EcoRV and BamHI to release a EcoRV-BamHI fragment which contains a portion of the 35S promoter (bp 7340-7433); pCGN638 is digested with HindIII and EcoRV to release a HindIII-EcoRV fragment containing a different portion of the 35S promoter (bp 6493-7340). These two fragments are ligated into pCGN986 which has been digested with HindIII and BamHI to remove the HindIII-BamHI fragment containing the 35S-promoter; this ligation produces pCGN639 which contains the backbone and *tm-1* 3' region from pCGN986 and the two 35S promoter fragments from pCGN164 and pCGN638. pCGN638 is digested with EcoRV and DdeI to release a fragment of the 35S promoter (bp 7070-7340); the fragment is treated with the Klenow fragment of DNA polymerase I to create blunt ends, and is ligated into the EcoRV site of pCGN639 to produce pCGN2113 having the fragment in the proper orientation.

### e. Construction of pCGN1761

pCGN2113 is digested with EcoRI and the plasmid is ligated in the presence of a synthetic DNA adaptor containing an XbaI site and a BamHI site (the adaptor contains EcoRI sticky ends on either end, but the adjacent bases are such that an EcoRI site is not reconstructed at this location) to produce pCGN2113M. pCGN2113M is digested to completion with SacI and then subjected to partial digestion with BamHI. This DNA is then treated with T4 DNA polymerase to create blunt ends and an EcoRI linker is ligated into the bluntended plasmid. After transformation a plasmid clone which contains a unique EcoRI site between the promoter and the intact *tm-1* 3' region is selected and designated pCGN1761.

The plasmid pBSLP5'PR1 is digested with EcoRI and the approximately 200 bp fragment containing the lytic peptide gene and PR-1a signal/leader is purified from a low-gelling temperature agarose gel. This fragment is ligated into pCGN1761 which is digested with EcoRI and treated with CIAP and also purified on low-gelling temperature agarose. The ligation reaction is used to transform *E*. *coli* and transformants are selected and then screened for the insert. Two types of transformants are recovered which differ in the orientation of the lytic peptide gene. One type, in which the gene is in the "sense" orientation with respect to the promoter, is selected and several plasmids are verified for the correct construct by DNA sequencing. One plasmid, which has the correct orientation and the anticipated DNA sequence, is designated pBS2X35SLytPep and used in further experiments.

### D. Subcloning the 2X35S CaMV/lytic peptide expression cassette into a binary vector

The expression cassette is subcloned into the binary plant transformation vector pCGN1540 containing the left and right T-DNA borders of *A. tumefaciens* octopine Ti-plasmid pTiA6 (Currier and Nester, 1976), the gentamycin resistance gene of pPHiJI (Hirsch and Beringer, 1984), an *A. rhizogenes* Ri plasmid origin of replication from pLJB11 (Jouanin et al., 1985), the *mas* promoter region an *mas* 3' region of pTiA6 with the kanamycin resistance gene of Tn5 (Jorgensen et al., 1979) a ColEl origin of replication from pBR322 (Bolivar et al., 1977), and a *lacZ'* screenable marker gene from pUC18 (Norrander et al., 1983). The backbone of pCGN1540, containing the gentamycin resistance gene and the Ri and ColEl origins, is derived from pCGN1532 (see below). The Ti borders and plant selectable marker gene (*mas 5'-kan-mas*3'), are from pCGN1537; the plant selectable marker cassette is in turn taken from pCGN1536, while the right border and the *lac*Z' fragments are derived from pCGN565RBx2X, and the left border is derived from pCGN65.

### a. pCGN1532 construction

The 3.5 kb EcoRI-PstI fragment containing the gentamycin resistance gene is removed from pPhlJI (Hirsch and Beringer, 1984) by EcoRI-PstI digestion and cloned into EcoRI-PstI digested pUC9 (Vieira and Messing, 1982) to generate pCGN549. HindIII-PstI digestion of pCGN549 yields a 3.1 kb fragment bearing the gentamycin resistance gene, which is made blunt ended by the Klenow fragment of DNA polymerase I and cloned into PvuII digested pBR322 (Bolivar et al., 1977) to create pBR322Gm. pBR322Gm is digested with DraI and SphI, treated with Klenow enzyme to create blunt ends, and the 2.8 kb fragment cloned into the Ri origin containing plasmid pLJbB11 1 (Jouanin et al., 1985) which has been digested with ApaI and made blunt ended with Klenow enzyme, creating pLHbB 11Gm. The extra ColEl origin and the kanamycin resistance gene are deleted from pLHbB 11GM by digestion with BamHI followed by self closure to create pGMB 11. The HindII site of pGmB 11 is deleted by HindII digestion followed by treatment with Klenow enzyme and self closure, creating pGmB 11-H. The PstI site of pGmB 11-H is deleted by PstI digestion followed by treatment with Klenow enzyme and self closure, creating pCGN1532.

### b. pCGN1536 construction

The 5.4 kb EcoRI fragment is removed from pVK232 (Knauf and Nester, 1982) by EcoRI digestion and cloned into EcoRI digested pACYC184 (Chang and Cohen, 1978) to create pCGN14. The 1434 bp ClaI-SphI fragment of pCGN14, containing the *mas* 5' region (bp20128-21562 according to numbering of Barker et al., 1983) is cloned into AccI-SphI digested pUC19 (Yanisch-Perron et al., 1985) to generate pCGN50. A 746 bp EcoRV-NaeI fragment of the *mas* 5' region is replaced by an XhoI site by digesting pCGN40 with EcoRV and NaeI followed by ligation in the presence of a synthetic Xho I linker DNA to create pCGN1036. The 765 bp SstI-HindIII fragment (bp 18474-19239) of pCGN14, containing the *mas* 3' region, is cloned into SstI-HindIII digested pUC18 (Norrander et al., 1983) to yield pCGN43. The HindIII site of pCGN43 is replaced with an EcoRI site by digestion with HindIII, blunt ending with Klenow enzyme, and ligation of synthetic EcoRI linker DNA to create pCGN1034. The 767 bp EcoRI fragment of pCGN 1034 is cloned into EcoRI-digested pCGN1036 in the orientation that places bp 19239 of the *mas* 3' region proximal to the *mas* 5' region to create pCGN1040. pCGN1040 is subjected to partial digestion with SstI, treated with T4 DNA polymerase to create blunt ends, and ligated in the presence of synthetic XhoI linker DNA; a clone is selected in which only the SstI site at the junction of bp 18474 and vector DNA (constructed in pCGN43 and carried into pCGN1040) is replaced by an XhoI site to generate pCGN1047. pCGN565 [see above] is digested with EcoRI and HindIII, treated with Klenow enzyme to create blunt ends, and ligated in the presence of synthetic Xhol linker DNA to create pCGN1003; this recreates the EcoRI site adjacent to the XhoI linker. pCGN1003 is digested with EcoRI, treated with Klenow enzyme to create blunt ends, and ligated in the presence of synthetic PstI linker DNA to create pCGN1007. The 1.5 kb XhoI fragment of pCGN1047, containing the *mas* 5' region and the *mas* 3' region with a multiple cloning site between, is cloned into XhoI digested pCGN1007 to construct pCGN1052.

A portion of the multiple cloning site of pCGN1052 is deleted by digestion with XbaI and SstI, treated with Klenow enzyme to make blunt ends, and ligated to generate pCGN1052δXS. The 1 kb EcoRI-SmaI fragment of pCGN550 [pCGN783 description], containing the 1 ATG-kanamycin resistance gene, is cloned into EcoRI-SmaI digested Bluescript M13-KS (Stratagene, Inc.) to create pBSKm; this plasmid contains an M13 region allowing generation of single stranded DNA. Single stranded DNA is generated according to the supplier's recommendations, and in vitro mutagenesis is performed (Adelman et al., 1983) using a synthetic oligonucleotide (SEQ ID NO:14) to alter a PstI site within the kanamycin resistance gene and make it undigestable, creating pCGN1534. pCGN1534 is digested with SmaI and ligated in the presence of synthetic EcoRI linker DNA to generate pCGN1535. The 1 kb EcoRI fragment of pCGN1536 is cloned into EcoRI digested pCGN1052δXS to create the *mas5'-kan mas3'* plant selectable marker cassette pCGN1536.

### c. pCGN565RAx2X construction

pCGN451 [pCGN783 description] is digested with HpaI and ligated in the presence of synthetic SphI linker DNA to generate pCGN55. The XhoI-SphI fragment of pCGN55 (bp13800-15208, including the right border, of *A*. *tumefaciens* T-DNA; Barker et al., 1983) is cloned into SalI-SphI digested pUC19 (Yanisch-Perron et al., 1985) to create pCGN60. The 1.4 kb HindIII-BamHI fragment of pCGN60 is cloned into HindIII-BamHI digested pSP64 (Promega, Inc.) to generate pCGN1039. pCGN1039 is digested with SmaI and NruI (deleting bp14273-15208; Barker et al., 1983) and ligated in the presence of synthetic BglII linker DNA creating pCGN1039δNS. The 0.47 kb EcoRI-HindIII fragment of pCGN1039δNS is cloned into Eco-RI-HindIII digested pCGN565 [described in pCFN783 description] to create pCGN565RB. The HindIII site of pCGN565RB is replaced with an XhoI site by HindIII digestion, treatment with Klenow enzyme, and ligation in the presence of synthetic XhoI linker DNA to create pCGN565RB-H+X. pUC18 (Norrander et al., 1983) is digested with HaeII to release the *lac*Z' fragment, treated with Klenow enzyme to create blunt ends, and the *lacZ'*-containing fragment ligated into pCGN565RB-H+X, which has been digested with AccI and SphI and treated with Klenow enzyme, in such an orientation that the *lacZ'* promoter is proximal to the right border fragment; this construct, pCGN565RBx2x is positive for *lacZ'* expression when plated on an appropriate host and contains bp 13990-14273 of the right border fragment (Barker et al., 1983) having deleted the AccI-SphI fragment (bp 13800-13990).

### d. pCGN65 construction

pCGN501 is constructed by cloning a 1.85 kb EcoRI-XhoI fragment of pTiA6 (Currier and Nester, 1976) containing bases 13362-15208 (Barker et al., 1983) of the T-DNA (right border), into EcoRI-SalI digested M13mp9 (Vieira and Messing, 1982). PCGN502 is constructed by cloning a 1.6 kb HindIII-SmaI fragment of pTiA6, containing bases 602-2212 of the T-DNA (left border), into HindIII-SmaI digested M13mp9. pCGN501 and pCGN502 are both digested with EcoRI and HindIII and both T-DNA-containing fragments cloned together into HindIII digested pUC9 (Vieira and Messing, 1982) to yield pCGN503, containing both T-DNA border fragments. pCGN503 is digested with HindIII and EcoRI and the two resulting HindIII-EcoRI fragments (containing the T-DNA borders) are cloned into EcoRI digested pHC79 (Hohn and Collins, 1980) to generate pCGN518. The KpnI-EcoRI fragment from pCGN518, containing the left T-DNA border, is cloned into KpnI-EcoRI digested pCGN565 to generate pCGN580. The BamHI-BglII fragment of pCGN580 is cloned into the BamHI site of pACYC184 (Chang and Cohen, 1978) to create pCGN51. The 1.4 kb BamHI-SphI fragment of pCGN60 (see pCGN65x2X section above) containing the T-DNA right border fragment, is cloned into BamHI-SphI digested pCGN51 to create pCGN65.

### e. pCGN1537 construction

pCGN65 is digested with KpnI and XbaI, treated with Klenow enzyme to create blunt ends, and ligated in the presence of synthetic BglII linker DNA to create pCGN65δKX. pCGN65δKX is digested with Sail, treated with Klenow enzyme to create blunt ends, and ligated in the presence of synthetic XhoI linker DNA to create pCGN65δKX-S+X. The 728 bp BglII-Xhol fragment of pCGNRBx2X, containing the T-DNA right border piece and the *lacZ'* gene, is cloned into BglII-XhoI digested pCGN65δKX-S+X, replacing pCGN65x2X. The ClaI fragment pCGN65x2X is deleted and replaced with an XhoI linker by digesting with ClaI, treated with Klenow enzyme to create blunt ends, and ligated in the presence of synthetic XhoI linker DNA to create pCGN65δ2XX. pCGN65δ2XXis digested with BglII and fused with BglII digested pCGN549 (see pCGN1532 section above) to create pCGN1530 which contains both plasmid backbones. pCGN1530 is digested with XhoI and religated, then a gentamycin-resistant chloramphenicol-sensitive clone is chosen which has deleted the pACYC184-derived backbone, creating pCGN1530A. The 2.43 kb XhoI fragment of pCGN1536, containing the *mas5'-kan-mas3'* cassette, is cloned into XhoI digested pCGN1530A to create pCGN1537.

### f. Final assembly of pCGN1540

The BglII fragment of pCGN1537, containing the plant selectable marker gene and the *lacZ'* screenable marker gene (with multiple cloning site), all between the T-DNA borders, is cloned into BamHI digested pCGN1532. A clone of the orientation bearing the T-DNA right border adjacent to the pBR322 origin of replication is designated pCGN1539, and the orientation bearing the T-DNA right border adjacent to the Ri plasmid origin of replication is designated pCGN1540. This binary vectors have several advantageous features, including a minimal amount of DNA between the T-DNA borders, high stability in *Agrobacterium* hosts, high copy number in *E*. *coli* hosts, and a blue/white screen with multiple restriction sites for ease of cloning target DNA.

The vector has been deposited into the ATCC (ATCC 40586). The XbaI fragment of pBS2X35SLytPep, which contains the lytic peptide gene, is subcloned into the XbaI site of pCGN1540 in such a way that the promoter fragment is proximal to the plant selectable marker gene. This plasmid is designated pBSBin2X35SLytPep and is selected for further experiments.

### E. Transformation of Agrobacterium

Binary vectors are transformed into *A*. *tumefaciens* strain LB4404 by the following method. The *Agrobacterium* strain is grown at 30°C overnight in 5 ml of LBMG medium (50 % L broth, 50 % mannitol-glutamate broth (Garfinkel and Nester, 1980). The 5 ml culture is added to 250 ml of LBMG and shaken vigorously until the culture density reaches an OD=0.6 at 600 nm wavelength. The cells are then collected by centrifugation at 8000 X g and resuspended in 5 ml of LBMG. 200 µl of cells are added to 0.2 to 1 µg of binary plasmid DNA in LBMG and the mix is frozen immediately in a dry ice/ethanol bath. After 5 minutes the tube is placed in a 37°C water bath for 5 minutes and then 2 ml of LBMG is added. The suspension is kept in a 30°C water bath for 2 to 3 hours and then the cells are collected by centrifugation. The cells are resuspended in a minimal volume of LBMG and then plated on selective media (LBMG plates with 100 µg/ml gentamycin). Colonies appear after 2 to 3 days at 30°C.

The plasmid pBSBin2X35SLytPep is transformed into *Agrobacterium* and positive colonies are selected and verified by Southern blot analysis and used for plant transformation experiments.

### Example 8: Stable Transformation and Regeneration of Transgenic Plants

Plant tissue is transformed with the vectors described above by any technique known in the art. Such methods used for transfer of DNA into plant cells include, for example, the direct infection of or co-cultivation of plants, plant tissue or cells with *A*. *tumefaciens* (Horsch et al., 1985; Marton, 1984), treatment of protoplasts with exogenous DNA by methods such as those described in Paszkowski et al. (1984); EP 164 575; Shillito et al. (1985); Potrykus et al. (1985); Lörz et al. (1985); Fromm et al. (1987); GB 2,140,822; and Negrutiu et al. (1987); incubation with polyethylene glycol (PEG) (Negrutiu et al., 1987); micro-injection [Reich et al. (1986a and b)]; microprojectile bombardment (Klein et al., 1987).

### A. Leaf Disk Transformation of Tobacco

*Agrobacterium* are grown 18 to 24 hours in glutamate salts media adjusted to pH 5.6 and supplemented with 0.15 % mannitol, 50 µg/ml kanamycin, 50 µg/ml spectinomycin and 1 mg/ml streptomycin before they are diluted to an OD600 of 0.2 in the same media without the antibiotics. The bacteria are then grown for three to five hours before dilution to an OD600 of 0.2 to 0.4 for inoculation of discs of 5 to 7 mm punched from leaves of *Nicotiana tabacum* cv. *xanthi* that have been grown aseptically in GA7 containers, following a modification of the method of Horsch et al. (1985).

The leaf disks are maintained on 0.7 % agar containing Murashige and Skoogs major and minor salts (MS), 1 mg/l benzyladenine and 1 mg/ml α-naphthaleneacetic acid for two days before transfer to the same media containing 50 µg/ml kanamycin, 100 µg/ml carbenicillin and 100 µg/ml mefoxin. Shoots which form on the discs are excised and propagated until six plantlets are obtained by subculturing the shoot tips on MS media containing 50 µg/ml kanamycin in GA7 containers.

The plantlets are rooted on medium containing no hormones and 50 µg/ml kanamycin, transferred to soil and hardened in a phytotron before transfer to the greenhouse for induction treatment with chemical regulators. At flowering time flowers are induced to selfpollinate. Seeds are harvested following maturation.

### B. Production of Transgenic Tobacco Callus and Plants

*Agrobacterium* is used to transform callus forming from the leaf disks (A). Callus forming on kanamycin-containing MSBN selection medium is maintained on a callus growth medium comprised of MS major, minor salts and Fe-EDTA (Gibco # 500-1117; 4.3 g/l), MS vitamins, 100 mg/l myo-inositol, 20 g/l sucrose, 2 mg/l naphthaleneacetic acid and 0.3 mg/l kinetin.

The callus can be used to regenerate transgenic plants by transferring callus pieces to MSBN medium and following methods described in A.

### C. Transformation of Carrot

*Agrobacterium* are grown as described in A. The bacteria, diluted to an OD600 of 0.2 to 0.4, are then used for inoculation of discs cut from surface sterilized carrots.

To surface sterilize the carrots they are peeled and then soaked 20 minutes in a 10 % solution of chlorox. The carrots are rinsed with sterile water, sliced into 5 mm pieces and placed basal side up onto water agar. 20 to 50 µl of bacteria are then applied to the upper surface of the discs.

### D. Transformation of Sunflower

*Agrobacterium* are grown as described in A. The bacteria, diluted to an OD600 of 0.2 to 0.4, are then used for inoculation of stems of sunflower plants prepared as follows:

Sunflower seeds are soaked 10 min in 10 % captan followed by 10 min in 10 % chlorox and rinsing with sterile water. The seed coats are removed and the seeds are germinated on 0.7 % water agar in the dark for three days, after which they are placed into a labline incubator set at 23°C with a 12 hour day and night. The seedlings are grown for one week before decapitation and inoculation of the bacteria onto the cut stem surface.

### E. Transformation of Tomato

*Agrobacterium* are grown as described in A. The bacteria, diluted to an OD600 of 0.2 to 0.4, are then used for inoculation of stems of tomato seedlings prepared as follows:

Tomato seeds are soaked 20 min in 10 % chlorox and rinsed with sterile water. The seeds are germinated on 0.7 % water agar in the dark for three days, after which they are placed into a labline incubator set at 23°C with a 12 hour day and night. The seedlings are grown for one week before decapitation and inoculation of the bacteria onto the cut stem surface.

### F. Transformation of Cotton

*Agrobacterium* are grown as described in A. The bacteria, diluted to an OD600 of 0.2 to 0.4, are then used for inoculation of cotton cotyledons prepared as follows:

The cotton seeds are soaked 20 min in 10 % chlorox and rinsed with sterile water. The seeds are germinated on 0.7 % water agar in the dark. The seedlings are grown for one week before inoculation of the bacteria onto the cotyledon surface.

### G. Preparation of a Special Type of Callus of Zea mays, Elite Inbred line Funk 2717

*Zea mays* plants of the inbred line Funk 2717 are grown to flowering in the greenhouse, and self pollinated. Immature ears containing embryos approximately 2 to 2.5 mm in length are removed from the plants and sterilized in 10 % Clorox solution for 20 minutes. Embryos are aseptically removed from the kernels and plated with the embryo axis downwards on OMS medium containing 0.1 mg/l 2,4-D, 6 % (w/v) sucrose and 25 mM L-proline solidified with 0.24 % (w/v) Gelrite® (initiation medium). After two weeks' culture in the dark at 27°C, the callus developing on the scutellum is removed from the embryo and plated on B5 medium, (Gamborg et al., 1968), containing 0.5 mg/l 2,4-D and solidified with 0.24 % (w/v) Gelrite® . The callus is subcultured every two weeks to fresh medium. After a total of eight weeks after placing the embryos on the initiation medium, the special type of callus is identified by its characteristic morphology. This callus is subcultured further on the same medium. After a further period of two months, the callus is transferred to, and serially subcultured on, N6 medium containing 2 mg/l 2,4-D and solidified with Gelrite® .

### H. Preparation of a Suspension Culture of Zea mays, Elite Inbred Funk 2717

The callus described in G is subcultured for a total of at least six months. The type of callus chosen for subculture is relatively non-mucilaginous, granular and very friable, such that it separated into small individual cell aggregates upon placing into liquid medium. Cultures containing aggregates with large, expanded cells are not retained. Approximately 500 mg aliquots of the special callus of *Zea mays* elite inbred funk 2717 are placed into 30 ml of N6 medium containing 2 mg/l 2,4-D in 125 ml Delong flasks. After one week of culture at 26°C in the dark on a gyratory shaker (130 rpm, 2.5 cm throw), the medium is replaced with fresh medium. The suspensions are again subcultured in this way after another week. At that time, the cultures are inspected, and those which do not show large numbers of expanded cells are retained. Suspension cultures containing aggregates with large, expanded cells are discarded. The preferred tissue consists of densely cytoplasmic dividing cell aggregates which has a characteristically smoother surface than the usual type of cell aggregates. The cultures retained have at least 50 % of the cells represented in these small aggregates. This is the desired morphology. These suspensions also have a rapid growth rate, with a doubling time of less than one week. The suspension cultures are subcultured weekly by transferring 0.5 ml PCV (packed cell volume: settled cell volume in a pipette) into 25 ml of fresh medium. After four to six weeks of subculture in this fashion, the cultures increase two- to three-fold per weekly subculture. Cultures in which more than 75 % of the cells are of the desired morphology are retained for further subculture. The lines are maintained by always choosing for subculture the flask whose contents exhibit the best morphology. Periodic filtration through 630 µm pore size stainless steel sieves every two weeks is used in some cases to increase the dispersion of the cultures, but is not necessary.

### I. Preparation of Protoplasts from Suspension Cultures of Zea mays

1 to 1.5 ml PCV of the suspension culture cells prepared as in H are incubated in 10 to 15 ml of a filter-sterilized mixture consisting of 4 % (w/v) cellulase RS with 1 % (w/v) Rhozyme in KMC (8.65 g/l KCl, 16.47 g/l MgCl₂. 6 H₂O and 12.5 g/l CaCl₂. H₂O, 5 g/l MES, pH 5.6) salt solution. Digestion is carried out at 30°C on a slow rocking table for a period of 3 to 4 hours. The preparation is monitored under an inverted microscope for protoplast release. The protoplasts which are released are collected as follows: The preparation is filtered through a 100 µm mesh sieve, followed by a 50 µm mesh sieve. The protoplasts are washed through the sieves with a volume of KMC salt solution equal to the original volume of enzyme solution. 10 ml of the protoplast preparation is placed in each of several disposable plastic centrifuge tubes, and 1.5 to 2 ml of 0.6 M sucrose solution (buffered to pH 5.6 with 0.1 % (w/v) morpholinoethane sulfonic acid (MES and KOH)) layered underneath. The tube is centrifuged at 60 to 100 x g for 10 minutes, and the protoplasts banding at the interface collected using a pipette and placed in a fresh tube. The protoplast preparation is resuspended in 10 ml of fresh KMC salt solution, and centrifuged for five minutes at 60 to 100 x g. The supernatant is removed and discarded, and the protoplasts resuspended gently in the drop remaining, and then 10 ml of a 13/14 strength KMC solution gradually added. After centrifuging again for five minutes, the supernatant is again removed and the protoplasts resuspended in a 6/7 strength KMC solution. An aliquot is taken for counting, and the protoplasts again sedimented by centrifugation. The protoplasts are resuspended at 10⁷ per ml in KM-8p medium or in 0.5 M mannitol containing 6 mM MgCl₂ or other suitable medium for use in transformation.

### J. Transformation of Zea mays Protoplasts by Electroporation

a. All steps except the heat shock are carried out at room temperature (22 to 28°C). The protoplasts are resuspended in the last step of I in 0.5 M mannitol containing 0.1 % (w/v) MES and 6 mM MgCl₂. The resistance of this suspension is measured in the chamber of a Dialog Electroporator (DIA-LOG GmbH, D-4000 Düsseldorf 13, Germany) and adjusted to 1 to 1.2 kΩ using a 300 mM MgCl₂ solution. The protoplasts are heat-shocked by immersing the tube containing the sample in a water bath at 45°C for five minutes, followed by cooling to room temperature on ice. 4 g of linearized plasmid containing a plant-selectable hygromycin resistance gene such as described by Rothstein et al. (1987), or chimeric gene constructs and 20 µg of calf thymus carrier DNA are added to aliquots of 0.25 ml of this suspension. 0.125 ml of a 24 % (w/v) PEG solution (MW 8000) in 0.5 M mannitol containing 30 mM MgCl₂ are added to the protoplasts. The mixture is mixed well but gently, and incubated for 10 minutes. The sample is transferred to the chamber of the electroporator and samples pulsed three times at 10 second intervals, at initial voltages of 1500, 1800, 2300 or 2800 Vcm⁻¹, and an exponential decay time of 1 µsec.

The protoplasts are cultured as follows. The samples are plated in 6 cm petri dishes at room temperature. After a further 5 to 15 minutes, 3 ml of KM-8p medium containing 1.2 % (w/v) SeaPlaque agarose and 1 mg/l 2,4-D are added. The agarose and protoplasts are mixed well and the medium is allowed to gel.

b. a is repeated with one or more of the following modifications:
(1) The resistance of the protoplast preparation is adjusted to 0.5 to 0.7 kΩ.
(2) The PEG used is PEG with a molecular weight of 4000.
(3) No PEG is added, or one-half volume of 12 % (w/v) PEG is added.
(4) The pulses are applied at intervals of three seconds.
(5) The protoplasts are plated after the electroporation in dishes placed on a plate cooled to a temperature of 16°C.
(6) The protoplasts are placed in tubes after the electroporation step, washed with 10 ml of 6/7 strength KMC solution or with W5 solution (comprised of 380 mg/l KCl, 18.375 g/l CaCl₂. 2 H₂O, 9 g/l NaCl; 9 g/l glucose, pH 6.0), then collected by centrifugation at 60 x g for 10 minutes, resuspended in 0.3 ml of KM medium, and plated as in a.
(7) The calf thymus carrier DNA is not added.

### K. Transformation of Zea mays Protoplasts by Treatment with PEG

a. The protoplasts are resuspended at the last step of I in a 0.5 M mannitol solution containing 12 to 30 mM MgCl₂. A heat shock of 45°C for five minutes is given as described in J. The protoplasts are distributed in aliquots for transformation in centrifuge tubes, 0.3 ml of suspended protoplasts per tube. During the next 10 minutes the following are added: DNA (as for J) and PEG solution (MW 6000, 40 % (w/v); containing 0.1 M Ca(NO₃)₂ and 0.4 M mannitol; pH 8 to 9 with KOH) to give a final concentration of 20 % PEG. The aliquots are incubated for 30 minutes with occasional gentle shaking, and then the protoplasts are placed in petri dishes (0.3 ml original protoplast suspension per 6 cm diameter dish) and cultured as described in J.

b. a is repeated and the protoplasts are washed after 30 minutes of incubation in the PEG solution of a by adding 0.3 ml of W5 solution five times at two- to three-minute intervals. The protoplast suspension is centrifuged, the supernatant removed, and the protoplasts are cultured as for Example J.a.

c. a and b are repeated with the modification that the final concentration of PEG is between 13 and 25 % (w/v).

### L. Regeneration of Callus From Protoplasts

The plates containing the protoplasts in agarose are placed in the dark at 26°C. After 14 days, colonies arise from the protoplasts. The agarose containing the colonies is transferred to the surface of a 9 cm diameter petri dish containing 30 ml of N6 medium containing 2 mg/12,4-D, solidified with 0.24 % w/v Gelrite®. This medium is referred to as 2N6. The callus is cultured further in the dark at 26°C and callus pieces subcultured every two weeks onto fresh solid 2N6 medium.

### M. Selection of Transformed Callus of Zea mays

L is repeated with the modification that 100 mg/l or 200 mg/l hygromycin B is added to the 2N6 medium in order to select for transformed cells.

### N. Regeneration of Corn Plants

a. Callus is placed on 2N6 medium for maintenance and on ON6 (comprising N6 medium lacking 2,4-D) and N61 medium (comprising N6 medium containing 0.25 mg/l 2,4-D and 10 mg/l kinetin) to initiate regeneration. Callus growing on ON6 and N61 media is grown in the light (16 hours/day light of 840 to 8400 lx from white fluorescent lamps). Callus growing on N61 medium is transferred to ON6 medium after two weeks, as prolonged time on N61 medium is detrimental. The callus is subcultured every two weeks even if the callus is to be transferred again on the same medium formulation. Plantlets appear in about four to eight weeks. Once the plantlets are at least 2 cm tall, they are transferred to ON6 medium in GA7 containers. Roots form in two to four weeks, and when the roots look well-formed enough to support growth, the plantlets are transferred to soil in peat pots, under a light shading for the first four to seven days. It is often helpful to invert a clear plastic cup over the transplants for two to three days to assist hardening off. Once the plants are established, they are treated as normal corn plants and grown to maturity in the greenhouse. In order to obtain progeny plants are self pollinated or crossed with wild type.

b. a is repeated with the modification that 100 mg/l or 200 mg/l hygromycin B is added to the medium used to maintain the callus.

### O. Preparation of Embryogenic Suspensions from Tissue of Dactylis glomerata L. (Orchardgrass)

a. Embryogenic callus is initiated from basal sections of the youngest leaves of greenhouse-grown orchardgrass plants as described by Hanning and Conger (1982). The leaves are surface sterilized by immersion in a 1:10 dilution of Clorox solution (5.25 % (w/v) sodium hypochlorite; The Clorox Company, Oakland, Ca.) for about 10 minutes and then cut aseptically into small segments of 1 to 5 mm in length or in diameter. These segments are plated on sterile SH-30 medium containing 0.8 % (w/v) agarose as a gelling agent. Callus and/or embryogenic structures appear within 2 to 6 weeks after plating, upon culture at about 25°C. Embryogenic callus is maintained by subculturing onto fresh SH-30 medium every 2 to 4 weeks and culturing in the dark at 25°C.

b. Embryogenic suspension cultures are initiated by placing approximately 0.5 g fresh weight of embryogenic callus into 50 ml of liquid medium described by Gray and Conger (1985) containing 45 µM dicamba and 4 g/liter casein hydrolysate. The suspension cultures are grown at 27°C under a 16 hours light (3300 lx), 8 hours dark photoperiod on a gyratory shaker at about 130 rpm in 125 ml Delong flasks sealed with a metal cap and parafilm® . After approximately four weeks the large clumps are allowed to settle for about 30 seconds and 10 ml aliquots of the supernatant medium containing small cell clusters are removed and transferred to 50 ml of fresh medium. This process is repeated every 3 to 4 weeks using the most successful cultures as judged by smaller clump size and better quality based on the presence of small, cytoplasmic cells. After 5 to 8 transfers the suspensions are essentially free of non embryogenic cells and the majority of the embryogenic cell clusters are quite small (150 to 2000 µm).

### P. Isolation and Purification of D. glomerata L. Protoplasts

Protoplasts are prepared from embryogenic suspension cultures of O by aseptically filtering the cells on a Nalgene® 0.2 µm filter unit and then adding 0.5 g fresh weight cells to each 12.5 ml of protoplast enzyme mixture in a petri dish. The enzyme mixture consists of 2 % (w/v) Cellulase RS, 7 mM CaCl₂ · H₂O, 0.7 mM NaH₂PO₄ · H₂O, 3 mM MES (pH 5.6), glucose (550 mOs/kg H₂O of pH 5.6), and is filter sterilized. The mixture is swirled on an orbital shaker at about 50 rpm in dim (< 400 lx) light for about 4 to 5 hours. The digest is then sieved through a stainless steel sieve (100 µm mesh size) and distributed into 12 ml centrifuge tubes which are centrifuged at about 60 to 100 x g for about 5 minutes. The protoplast-containing sediment is then washed three times with protoplast culture medium KM-8p adjusted to 550 mOs/kg H₂O with glucose. At this point a flotation step may be included for further purification of the protoplasts. In this case, the washed protoplasts are layered atop 10 ml of KM-8p culture medium adjusted 700 mOs/kg H₂O with sucrose. After centrifugation at 60 to 100 x g for about 10 minutes, protoplasts banding at the interface are collected using a fine pipette. Finally, the protoplasts are resuspended in 1 to 2 ml KM-8p culture medium and sieved through a stainless mesh screen (20 µm mesh size). The protoplasts released are collected and washed and resuspended in KM-8p medium for culture or in osmotically adjusted medium suitable for transformation.

### Q. D. glomerata L. Protoplast Culture and Growth of Callus

a. The purified protoplasts are plated at a density of about 5 x 10⁵ protoplasts per ml in KM-8p culture medium containing 1.3 % (w/v) SeaPlaque® agarose (FMC Corp., Marine Colloids Division, Rockland, Maine, USA) and 30 to 40 % (w/v) of conditioned medium (obtained from 3 to 4 week-old *D. glomerata* L. embryogenic suspension cultures by filtering the medium through a sterile Nalgene® 0.2 µm filter, making the medium 550 mOsm/kg H₂O by addition of glucose, and again filter sterilizing). The plates are then placed in the dark at a constant temperature of 28°C. After 10 to 14 days the agarose is cut into wedges and placed into 'bead culture' as described by Shillito et al. (1983) using 20 ml SH-45 suspension culture medium with 3 % (w/v) sucrose per 3 ml original agarose embedded culture. The plates are put on a platform shaker and agitated at about 50 rpm in light at 670 lx. New suspension cultures are formed as the colonies grow out of the agarose and release cells into the liquid medium. The resultant suspension cultured cells are plated onto agar-solidified SH-30 medium and placed in the dark at 25°C until callus is formed.

b. Protoplasts are cultured as described in a above except that the culture media contains no conditioned medium.

### R. Transformation of D. glomerata L. Protoplasts by Means of Electroporation

a. Immediately after purification of the protoplasts, electroporation is performed according to Shillito et al. (1985) using linearized plasmid. The protoplasts are resuspended after the last wash at a density of about 7 x 10⁶ protoplasts per ml in the electroporation buffer (0.4 M mannitol, 6 mM MgCl₂). The protoplasts are placed in 0.7 ml aliquots in 10 ml plastic centrifuge tubes. Plasmid DNA and sonicated calf thymus DNA (Sigma) to give final concentrations of 10 µg/ml and 50 µg/ml respectively is added to the tubes. Then 0.38 ml PEG solution [24 % (w/v) PEG 6000 in 0.4 M mannitol 30 mM MgCl₂, 0.1 % (w/v) MES (pH 5.6)] is added and the solution gently mixed. The protoplast suspension is transferred into the chamber of a Dialog® Electroporator and 10 pulses of 3250 V/cm initial voltage and exponential decay constant of 10 µsec applied at 30 sec intervals. The sample is removed from the chamber, and placed in a 10 cm diameter petri dish. 10 ml of KM-8p medium containing 1.2 % (w/v) SeaPlaque® agarose is added, the protoplasts distributed evenly throughout the medium, and the agarose allowed to gel.

b. a is repeated except that the initial voltage used is 3500 V/cm, 4000 V/cm, 5000 V/cm, 3000 V/cm, or 2500 V/cm.

c. a and b are repeated except that PEG of MW 4000 or PEG of MW 8000 is used.

d. a to c are repeated except that the final PEG concentration is between 10 % and 30 % (w/v).

### S. Transformation of D. glomerata L. Protoplasts by Treatment with PEG

a. PEG mediated direct gene transfer is performed according to Negrutiu et al. (1987). The DNA used is linearized plasmid.

The protoplasts are suspended following the last wash in 0.5 M mannitol containing 15 mM MgCl₂ at a density of about 2 x 10⁶ per ml. The protoplast suspension is distributed as 1 ml aliquots into 10 ml plastic centrifuge tubes. The DNA is added as described in R above, and then 0.5 ml of the PEG solution added (40 % (w/v) PEG 4000 in 0.4 M mannitol, 0.1 M Ca(NO₃)₂, pH 7.0). The solutions are mixed gently and incubated for 30 minutes at room temperature (about 24°C) for 30 minutes with occasional shaking. 1.4 ml of the wash solution is then added, and the contents of the tube gently mixed. The wash solution consists of 87 mM mannitol, 115 mM CaCl₂, 27 mM MgCl₂, 39 mM KCl, 7 mM Tris-HCl and 1.7 g/l myo-inositol, pH 9.0. Four further 1.4 ml aliquots of wash solution are added at 4 minute intervals, with mixing after each addition. The tube is then centrifuged at about 60 x g for about 10 minutes, and the supernatant discarded. The sedimented protoplasts are taken up in 1 ml KM-8p culture medium, and placed in a 10 cm petri dish. 10 ml of KM-8p medium containing 1.2 % (w/v) SeaPlaque® agarose is added. The protoplasts are evenly distributed throughout the medium, and the agarose allowed to gel.

b. a is repeated with one or more of the following modifications:
(1) The pH of the wash solution is adjusted to 5.6 or 7.0.
(2) The PEG used is PEG of MW 6000, PEG of MW 2000 or PEG of MW 8000.
(3) The wash medium consists of 154 mM NaCl, 125 mM CaCl₂, 5 mM KCl, 5 mM glucose, pH to 6.0 with KOH, of 0.2 M CaCl₂, 0.1 % (w/v) MES, pH 6.0 with KOH, or of 0.2 M CaCl₂, 7 mM Tris/HCl, pH 9.0 with KOH.

### T. Transformation of D. glomerata L. Protoplasts by Electroporation or PEG Treatment

Transformation is carried out as described in R or S, except that the protoplasts are treated at 45°C for about 5 minutes prior to distribution of the aliquots into tubes for transformation or after distribution of the aliquots, and before addition of the PEG.

### U. Selection of Transformed Colonies

a. The culture plates (petri dishes) containing the protoplasts from R to T are incubated for 10 days in the dark at about 25°C and then cut into 5 equal slices for 'bead cultures' (Shillito et al., 1983). Four of the slices are placed each into 20 ml SH-45 culture medium with 4 g/l casein hydrolysate and 20 µg/ml hygromycin B. The fifth slice is put into 20 ml of the same medium but without hygromycin B as a non-selected control. After 4 to 5 weeks the putative transformed protoplast-derived cell colonies growing in hygromycin B are cut out of the agarose and placed into a 19 mm petri dish with 2 ml of liquid SH-45 medium containing 20 µg/ml hygromycin B, which is agitated at about 50 rpm on an orbital shaker. After another 4 to 5 weeks all colonies which grow to make new suspensions are transferred into 125 ml Erlenmeyer flasks and grown in a manner similar to the parent suspension culture, except that 20 µg/ml hygromycin B is included in the medium.

The new suspensions are subcultured every 1 to 3 weeks using SH-45 medium containing 4 g/l casein hydrolysate and 20 µg/ml hygromycin B. Cells from these suspensions are also plated on solidified SH-30 medium containing 20 µg/ml hygromycin B and incubated at about 25°C in the dark. Calli grown from the plated cells are subcultured every two weeks onto fresh medium. The cells which grow in the presence of hygromycin B are presumed to be transformants.

b. Selection is carried out as described in a except that the protoplast-derived cell colonies growing in hygromycin B containing medium are placed on agar plates of SH-30 medium containing 20 µg/ml hygromycin B and incubated at about 25°C in the dark.

### V. Regeneration of Transformed D. glomerata L. Plants

a. *D. glomerata* L. callus (obtained as described in U) derived from protoplasts is grown on solidified SH-30 medium, and subcultured every two weeks. Any embryos which form are removed and plated on germination medium (SH-0) and placed in the light (3800 to 4600 lx). Germination of these embryos occurs in 1 to 4 weeks and the resultant plantlets are placed on SH-0 medium in the light to form root systems. They are moved into the greenhouse at the six to twelve leaf stage, and hardened off gradually.

b. Callus (obtained as described in U) derived from protoplasts is grown on SH-0 medium solidified with 0.24 % (w/v) Gelrite® in the light (3800 to 4600 lx), and subcultured every two weeks. The resultant plantlets are placed on a 1:1 mixture of SH-0 and OMS media solidified with a combination of 0.12 % (w/v) Gelrite® and 0.4 % (w/v) agar in the light to form root systems. They are moved to the greenhouse at the six to twelve leaf stage, and hardened off gradually.

c. Small plantlets are obtained as described above, and are placed on OMS medium solidified with 0.8 % (w/v) agar in the light to form root systems. They are moved to the greenhouse at the six to twelve leaf stage, and hardened off gradually.

d. Small plantlets are obtained as described above and are placed on a 1:1 mixture of SH-0 and OMS media solidified with a combination of 0.12 % (w/v) GelRite® and 0.4 % (w/v) agar in the light to form root systems. They are moved to the greenhouse at the six to twelve leaf stage, and hardened off gradually.

### Example 9: Development of transgenic T3 seed lines containing all three genes

Genotype designations for transgenic plants are used herein according to the following convention: the initial plant resulting from a transformation event and having grown from tissue culture is designated a T1 plant. Plants resulting from self pollination of the natural flowers of the T1 plant, are designated T2, having acquired a new genotype during the normal meiotic process. Likewise, seeds borne from self-pollination of the natural flowers of T2 plants (i.e. grown from T2 seed) are designated T3, etc.

Transgenic plants (T1) are grown to maturity. Flowers are allowed to self-pollinate and seed pods are collected after normal dessication. Seeds from each individual plant are collected and stored separately. Each seed lot is tested by genetic segregation analysis to determine the number of Mendelian loci bearing the kanamycin resistance trait. T2 seeds are surface-sterilized by multiple washing in 2 % hypochlorite containing 0.02 % (v/v) Tween-20, followed by rinses in sterile water. Approximately 150 of the seeds are placed on filter paper saturated with 0.2 X MS salts (Murashige and Skoog, 1962) containing 150 µg/ml kanamycin. Following germination and expansion of the cotyledons to approximately 5 mm, the ratio of normal-green (*kan-r*) versus bleached (*kan-s*) cotyledons is determined. Only those T2 seed lots exhibiting an approximately 3:1 (*kan-r:kan-s*) ratio are kept for further analysis; this segregation ratio is indicative of a single Mendelian locus bearing the kanamycin marker gene.

Four to ten plants are grown to maturity from each T2 seed lot (using the same conditions described above), and are allowed to self-pollinate. T3 seed collection, seed sterilization, and seed germination are as described above for the T2 seed. T3 seed lots in which 100 % of the tested seeds (n = 150) exhibit the kan-r phenotype are assumed to be homozgous for the trait (i.e. resulting from a homozygous T2 parent plant) and are kept for phenotypic analysis.

Conventional breeding techniques are then used to get all three genes into the same plant.

While the present invention has been described with reference to specific embodiments thereof, it will be appreciated that numerous variations, modifications, and embodiments are possible, and accordingly, all such variations, modifications and embodiments are to be regarded as being within the spirit and scope of the present invention.

### References

Adelman, J.P., Hayflick, J.S., Vasser, M., Seeburg, P.H., DNA 2:183-193 (1983)

Barker, R.F., Idler, K.B., Thompson, D.V., Kemp, J.D., Plant Molec. Biol. 2:335-350 (1983)

Bohlmann, H., Clausen, S., Behnke, S., Giese, H., Hiller, C., Reimann-Philipp, U., Schrader, G., Barkholt, V., Apel, K., EMBO J. 7:1559-1565 (1988)

Bolivar, F., Rodriguez, R.L. Greene, P.J., Betlach, M.C., Heyneker, H.L., Boyer, H.W., Crosa, J.H., Falkow, S., Gene 2:95-113 (1977)

Boller, T., Gehri, A., Mauch, F., Vögeli, U., Planta 157:22-31 (1983)

Bradford, M.M., Analyt. Biochem. 72:248-254 (1976)

Burri, M., Untersuchungen an einem Bioassay zur Hemmung von *Fusarium solani* f. sp. *pisi* durch pflanzliche Chitinasen und β-1,3-Glucanasen, Diplomarbeit, Basel (1989)

Chang, A.C.Y., Cohen., S.N., J. Bacteriol. 134:1141-1156 (1978)

Currier, T.C., Nester, E.W., J. Bacteriol. 126:157-165 (1976)

Dygert, S., Li, L.H., Florida, D., Thoma, J.A., Analyt. Biochem. 13:367-374 (1965)

Fromm, M., Callis, J., Taylor, L.P., Walbot, V., Methods in Enzymology 153:351-366 (1987)

Gamborg, O.L., Miller, R.A., Ojima, K., Exptl. Cell Res. 50:151-158 (1968)

Gardner, R.C., Howarth, A.J., Hahn, P., Brown-Luedi, M., Shepherd, R.J., Messing, J., Nucl. Acids Res. 9:2871-2888 (1981)

Garfinkel, D.J., Nester, E.W., J. Bacteriol. 144:732-743 (1980)

Gray, D.J., Conger, B.V., Plant Cell Tissue Organ Culture 4:123-133 (1985)

Hanning, G.E., Conger, B.V., Theor. Appl. Genet. 63:155-159 (1982)

Hirsch, P.R., Beringer, J.E., Plasmid 12:139-141 (1984)

Ho, S.N., Hunt, H.D., Horton, R.M., Pullen, J.K., Pease, L.R., Gene 77:51-59 (1989)

Hohn, B., Collins, J., Gene 11:291-298 (1980)

Horsch, R.B., Fry, J.E., Hoffmann, N.L., Eichholtz, D., Rogers, S.G., Fraley, R.T., Science 227:1229-1231 (1985)

Jamet, E., Fritig, B., Plant Mol. Biol. 6:69-80 (1986)

Jorgensen, R.A., Rothstein, S.J., Reznikoff, W.S., Molec. gen. Genet. 177:65-72 (1979)

Jouanin, L., Vilaine, F., D'Enfert, C., Casse-Delbart, F., Mol. Gen. Genet 201:370-374 (1985)

Kauffmann, S., Legrand, M., Geoffroy, P., Fritig, B., EMBO J. 6:3209-3212 (1987)

Klein, T.M., Wolf, E.D., Wu, R., Sanford, J.C., Nature 327:70-73 (1987)

Knauf, V.C., Nester, E.W., Plasmid 8:45-54 (1982)

Legrand, M., Kauffmann, S., Geoffroy, P., Fritig, B., Proc. Natl. Acad. Sci. USA, 84:6750-6754 (1987)

Lehrer, R.I., Szklarek, D., Ganz, T., Selsted, M.E., Infection and Immunity 52:902-904 (1986)

van Loon, L.C., Plant Mol. Biol. 4:11-116 (1985)

Lörz, H., Baker, B., Schell, J., Mol. Gen. Genet. 199:178-182 (1985)

Marton, L., in: Cell Culture and Somatic Cell Genetics of Plants 1:514-521 (1984)

Mauch, F., Mauch-Mani, B., Boller, T., Plant Phys. 88:936-942 (1988)

McCutcheon's Detergents and Emulsifiers Annual, MC Publishing Corp., Ringwood, New Jersey (1979)

Messing, J., Crea, R., Seeburg, P.H., Nucl. Acids Res. 9:309-321 (1981)

Molano, J., Durán, A., Cabib, E., Analyt. Biochem. 83:648-656 (1977)

Murashige, T., Skoog, F., Physiol. Plant. 15:473-497 (1962)

Negrutiu, I., Shillito, R., Potrykus, I., Biasini, G., Sala, F., Plant Mol. Biol. 8:363-373 (1987)

Norrander, J., Kempe, T., Messing, J., Gene 26:101-106 (1983)

Ogasawara, N., Tanaka, H., Yoshinaga, E., Hiraga, K., CA 79:133662v (1973)

Paszkowski, J., Shillito, R.D., Saul, M., Mandak, V., Hohn, T., Hohn, B., Potrykus, I., EMBO J. 3:2717-2722 (1984)

Potrykus, I., Paszkowski, J., Saul, M.W., Petruska, J., Shillito, R.D., Mol. Gen. Genet. 199:169-177 (1985)

Reich, T.J., Iyer, V.N., Haffner, M., Holbrook, L.A., Miki, B.L., Can. J. Bot. 64:1259-1267 (1986)

Reich, T.J., Iyer, V.N., Miki, B.L., Biotechnology 4:1001-1004 (1986)

Rothstein, S.J., Lahners, K.N., Lotstein, R.J., Carozzi, N.B., Jayne, S.M., Rice, D.A., Gene 53:153-161 (1987)

Sambrook, J., Fritsch, E.F., Maniatis, T., Molecular cloning, a laboratory manual, Cold Spring Harbor Laboratory, Cold Spring Harbor (1989)

Schlumbaum, A., Mauch, F., Vögeli, U., Boller, T., Nature 324:365-367 (1986)

Selsted, M.E., Harwig, S.S.L., Infection and Immunity 55:2281-2286 (1987)

Shillito, R.D., Paszkowski, J., Potrykus, I., Plant Cell Reports 2:244-247 (1983)

Shillito, R.D., Saul, M.W., Paszkowski, J., Müller, M., Potrykus, I., Biotechnology 3:1099-1103 (1985)

Shinshi, H., Mohnen, D., Meins, F., Proc. Natl. Acad. Sci. USA 84:89-93 (1987)

Shinshi, H., Wenzler, H., Neuhaus, J.-M., Felix, G., Hofsteenge, J., Meins, F., Proc. Natl. Acad. Sci. USA 85:5541-5545 (1988)

Sisely, Wood, Encyclopedia of Surface Active Agents, Chemical Publishing Co., New York (1980)

Terry, A.S., Poulter, L., Williams, D.H., Nutkins, J.C., Giovannini, M.G., Moore, C.H., Gibson, B.W., J. Biol. Chem. 263:5745-5751 (1988)

Thomashow, M.F., Nutter, R., Montoya, A.L., Gordon, M.P., Nester, E.W., Cell 19:729-739 (1980)

Vieira, J., Messing, J., Gene 19:259-268 (1982)

Yanisch-Perron, C., Vieira, J., Messing, J., Gene 33:103-119 (1985)

### Sequence listing

SEQ ID NO: 1
   SEQUENCE TYPE: Peptide
   SEQUENCE LENGTH: 22 Amino acids 
Gly Ile Gly Lys Phe Leu His Ser Ala Lys Lys Phe Gly Lys Ala
                5                   10                  15
Phe Val Gly Ile Met Asn Ser
                20
SEQ ID NO:2
   SEQUENCE TYPE: Nucleotide
   SEQUENCE LENGTH: 75 base pairs 
GAATTCGGGA TCGGCAAGTT CCTGCACAGC GCCAAGAAGT TCGGGAAGGC 50
CTTCGTGGGC ATCATGAACA GCTAA 75
SEQ ID NO:3
   SEQUENCE TYPE: Nucleotide
   SEQUENCE LENGTH: 75 base pairs 
GAATTCTTAG CTGTTCATGA TGCCCACGAA GGCCTTCCCG AACTTCTTGG 50
CGCTGTGCAG GAACTTGCCG ATCCC 75
SEQ ID NO:4
   SEQUENCE TYPE: Nucleotide
   SEQUENCE LENGTH: 38 base pairs TATCCCACTC TTGCCGTGCC GGGATCGGCA AGTTCCTG 38
SEQ ID NO:5
   SEQUENCE TYPE: Nucleotide
   SEQUENCE LENGTH: 38 base pairs CAGGAACTTG CCGATCCCGG CACGGCAAGA GTGGGATA 38
SEQ ID NO:6
   SEQUENCE TYPE: Nucleotide
   SEQUENCE LENGTH: 30 base pairs 
GATCGAATTC ATTCAAGATA CAACATTTCT 30
|EcoRI | PR-1a |
SEQ ID NO:7
   SEQUENCE TYPE: Nucleotide
   SEQUENCE LENGTH: 27 base pairs GAATTCTTAG CTGTTCATGA TGCCCAC 27
SEQ ID NO:8
   SEQUENCE TYPE: Nucleotide
   SEQUENCE LENGTH: 38 base pairs 
TATCCCACTC TTGCCGTGCC GGGATCGGCA AGTTCCTG 38
ATAGGGTGAG AACGGCACGG CCCTAGCCGT TCAAGGAC 38
| PR-1a | | lytic peptide 3 |
SEQ ID NO:9
   SEQUENCE TYPE: Nucleotide with corresponding protein
   SEQUENCE LENGTH: 1358 base pairs 
CCTCAAA TGG CTG CTA TCA CAC TCC TAG GAT TAC TAC TTG TTG 43
Met Ala Ala Ile Thr Leu Leu Gly Leu Leu Leu Val

CCA GCA GCA TTG ACA TAG CAG GGG CTC AAT CGA TAG GTG TTT 85
Ala Ser Ser Ile Asp Ile Ala Gly Ala Gln Ser Ile Gly Val

GCT ATG GAA TGC TAG GCA ACA ACT TGC CAA ATC ATT GGG AAG 127
Cys Tyr Gly Met Leu Gly Asn Asn Leu Pro Asn His Trp Glu

TTA TAC AGC TCT ACA AGT CAA GAA ACA TAG GAA GAC TGA GGC 169
Val Ile Gln Leu Tyr Lys Ser Arg Asn Ile Gly Arg Leu Arg

TTT ATG ATC CAA ATC ATG GAG CTT TAC AAG CAT TAA AAG GCT 211
Leu Tyr Asp Pro Asn His Gly Ala Leu Gln Ala Leu Lys Gly

CAA ATA TTG AAG TTA TGT TAG GAC TTC CCA ATT CAG ATG TGA 253
Ser Asn Ile Glu Val Met Leu Gly Leu Pro Asn Ser Asp Val

AGC ACA TTG CTT CCG GAA TGG AAC ATG CAA GAT GGT GGG TAC 295
Lys His Ile Ala Ser Gly Met Glu His Ala Arg Trp Trp Val
 
AGA AAA ATG TTA AAG ATT TCT GGC CAG ATG TTA AGA TTA AGT 337
Gln Lys Asn Val Lys Asp Phe Trp Pro Asp Val Lys Ile Lys

ATA TTG CTG TTG GGA ATG AAA TCA GCC CTG TCA CTG GCA CAT 379
Tyr Ile Ala Val Gly Asn Glu Ile Ser Pro Val Thr Gly Thr

CTT ACC TAA CCT CAT TTC TTA CTC CTG CTA TGG TAA ATA TTT 421
Ser Tyr Leu Thr Ser Phe Leu Thr Pro Ala Met Val Asn Ile

ACA AAG CAA TTG GTG AAG CTG GTT TGG GAA ACA ACA TCA AGG 463
Tyr Lys Ala Ile Gly Glu Ala Gly Leu Gly Asn Asn Ile Lys

TCT CAA CTT CTG TAG ACA TGA CCT TGA TTG GAA ACT CTT ATC 505
Val Ser Thr Ser Val Asp Met Thr Leu Ile Gly Asn Ser Tyr

CAC CAT CAC AGG GTT CGT TTA GGA ACG ATG CTA GGT GGT TTA 547
Pro Pro Ser Gln Gly Ser Phe Arg Asn Asp Ala Arg Trp Phe

CTG ATC CAA TTG TTG GGT TCT TAA GGG ACA CAC GTG CAC CTT 589
Thr Asp Pro Ile Val Gly Phe Leu Arg Asp Thr Arg Ala Pro

TAC TCG TTA ACA TTT ACC CCT ATT TCA GCT ATT CTG GTA ATC 631
Leu Leu Val Asn Ile Tyr Pro Tyr Phe Ser Tyr Ser Gly Asn

CAG GGC AGA TTT CTC TCC CCT ATT CTC TTT TTA CAG CAC CAA 673
Pro Gly Gln Ile Ser Leu Pro Tyr Ser Leu Phe Thr Ala Pro

ATG TGG TAG TAC AAG ATG GTT CAC GCC AAT ATA GGA ACT TAT 715
Asn Val Val Val Gln Asp Gly Ser Arg Gln Tyr Arg Asn Leu

TTG ATG CAA TGC TGG ATT CTG TGT ATG CTG CCC TCG AGC GAT 757
Phe Asp Ala Met Leu Asp Ser Val Tyr Ala Ala Leu Glu Arg

CAG GAG GGG CAT CTG TAG GGA TTG TTG TGT CCG AGA GTG GCT 799
Ser Gly Gly Ala Ser Val Gly Ile Val Val Ser Glu Ser Gly

GGC CAT CTG CTG GTG CAT TTG GAG CCA CAT ATG ACA ATG CAG 841
Trp Pro Ser Ala Gly Ala Phe Gly Ala Thr Tyr Asp Asn Ala

CAA CTT ACT TGA GGA ACT TAA TTC AAC ACG CTA AAG AGG GTA 883
Ala Thr Tyr Leu Arg Asn Leu Ile Gln His Ala Lys Glu Gly

GCC CAA GAA AGC CTG GAC CTA TTG AGA CCT ATA TAT TTG CCA 925
Ser Pro Arg Lys Pro Gly Pro Ile Glu Thr Tyr Ile Phe Ala

TGT TTG ATG AGA ACA ACA AGA ACC CTG AAC TGG AGA AAC ATT 967
Met Phe Asp Glu Asn Asn Lys Asn Pro Glu Leu Glu Lys His

TTG GAT TGT TTT CCC CCA ACA AGC AGC CCA AAT ATA ATA TCA 1009
Phe Gly Leu Phe Ser Pro Asn Lys Gln Pro Lys Tyr Asn Ile

ACT TTG GGG TCT CTG GTG GAG TTT GGG ACA GTT CAG TTG AAA 1051
Asn Phe Gly Val Ser Gly Gly Val Trp Asp Ser Ser Val Glu

CTA ATG CTA CTG CTT CTC TCG TAA GTG AGA TGT GAGCTGATGAG 1095
Thr Asn Ala Thr Ala Ser Leu Val Ser Glu Met

ACACTTGAAA TCTCTTTACA TACGTATTCC TTGGATGGAA AACCTAGTAA 1145
 
AAACAAGAGA AATTTTTTCT TTATGCAAGA TACTAAATAA CATTGCATGT 1195
CTCTGTAAGT CCTCATGGAT TGTTATCCAG TGACGATGCA ACTCTGAGTG 1245
GTTTTAAATT CCTTTTCTTT GTGATATTGG TAATTTGGCA AGAAACTTTC 1295
TGTAAGTTTG TGAATTTCAT GCATCATTAA TTATACATCA GTTCCATGTT 1345
TGATCAAAAA AAA 1358
SEQ ID NO:10
   SEQUENCE TYPE: Nucleotide
   SEQUENCE LENGTH: 8 bases CATCTGAATT CTCCCAACAA GTCTTCCC
SEQ ID NO:11
   SEQUENCE TYPE: Nucleotide
   SEQUENCE LENGTH: 42 bases AACACCTATC GATTGAGCCC CTGCTATGTC AATGCTGGTG GC
SEQ ID NO:12
   SEQUENCE TYPE: Nucleotide
   SEQUENCE LENGTH: 81 base pairs 
GGATCCGTTT GCATTTCACC AGTTTACTAC TACATTAAAA TGAGGCTTTG 50
TAAATTCACA GCTCTCTCTT CTCTACTATT T 81
SEQ ID NO:13
   SEQUENCE TYPE: Nucleotide
   SEQUENCE LENGTH: 14 base pairs GATCCGGAAT TCCG
SEQ ID NO:14
   SEQUENCE TYPE: Nucleotide
   SEQUENCE LENGTH: 17 bases

GAACTCCAGG ACGAGGC

## Claims (Claims for the following Contracting State(s): AT, BE, CH, DE, DK, FR, GB, GR, IT, LU, NL, SE)

1. An anti-pathogenic composition for controlling plant pathogens comprising an active ingredient together with one or more agriculturally acceptable carriers; wherein the active ingredient comprises
(a) one or more lytic peptides, which is not lysozyme, in combination with;
(b) one or more chitinases; and/or
(c) one or more β-1,3-glucanases
in a synergistically effective amount.

2. The composition of claim 1 wherein the lytic peptide is any natural or synthetic peptide or any functional derivative thereof that is capable of anti-pathogenically effective activity due to its ability to penetrate, lyse or otherwise impair the pathogen's cell membrane.

3. The composition of claim 2 wherein the lytic peptide is selected from the group consisting of mammalian defensins, cecropins, thionins, mellitins, insect defensins, magainins, attacins, dipteris, sapecins, cacrutins, xenopsins, or hybrids thereof.

4. The composition of any one of claims 1 to 3 wherein chitinase is present in the active ingredient at a relative concentration of about 1/10 c.

5. The composition of any one of claims 1 to 3 wherein chitinase is present in the active ingredient at a relative concentration of about 1/100 c.

6. The composition of any one of claims 1 to 3 wherein the β-1,3-glucanase is present in the active ingredient at a relative concentration of about 1/10 c.

7. The composition of any one of claims 1 to 3 wherein the β-1,3-glucanase is present in the active ingredient at a relative concentration of about 1/100 c.

8. The composition of any one of claims 1 to 3 wherein chitinase is present in the active ingredient at a relative concentration of about 1/10 c and the β-1,3-glucanase is present in the active ingredient at a relative concentration of about 1/10 c.

9. The composition of any one of claims 1 to 3 wherein chitinase is present in the active ingredient at a relative concentration of about 1/10 c and the β-1,3-glucanase is present in the active ingredient at a relative concentration of about 1/100 c.

10. The composition of any one of claims 1 to 3 wherein chitinase is present in the active ingredient at a relative concentration of about 1/100 c and the β-1,3-glucanase is present in the active ingredient at a relative concentration of about 1/100 c.

11. The composition of any one of claims 1 to 3 wherein chitinase is present in the active ingredient at a relative concentration of about 1/100 c and the β-1,3-glucanase is present in the active ingredient at a relative concentration of about 1/10 c.

12. The composition of claim 1, wherein one of the lytic peptides is synthetic peptide no. 3.

13. The composition of any one of claims 1 to 3 wherein the lytic peptides are present in a concentration from about 1 to about 200 ppm.

14. A method of controlling plant pathogens comprising applying to the pathogen or the locus thereof an anti-pathogenically effective amount of a composition comprising an active ingredient together with one or more agriculturally acceptable carriers; wherein the active ingredient comprises
(a) one or more lytic peptides which is not lysozyme, in combination with;
(b) one or more chitinases; and/or
(c) one or more β-1,3-glucanases in a synergistically effective amount.

15. The method according to claim 14, wherein the lytic peptide is used in combination with one or more activated chitinases; and/or one or more activated β-1,3-glucanases, which are already present in the plant to be protected using an external inducing stimulus.

16. A method of controlling plant pathogens by expressing in a transgenic plant one or more lytic peptides together with one or more chitinases; and/or one or more β-1,3-glucanases in a synergistically effective amount;
said method comprising the steps of
(a) preparing a transgenic plant comprising recombinant DNA sequences encoding one or more lytic peptides, which is not lysozyme, one or more chitinases; and/or one or more β-1,3-glucanases; and
(b) causing the transgenic plant to synthesize the compounds mentioned in step (a).

17. The method according to claim 16, wherein the chitinase(s) and/or the β-1,3-glucanase(s) are part of an inducible expression system comprising an inducible gene and an inducing regulator and wherein the expression of at least one lytic peptide is followed by treatment with an inducing regulator which induces the accumulation of at least one natural or foreign chitinase and/or one natural or foreign β-1,3-glucanase.

18. A transgenic plant comprising recombinant DNA sequences encoding
(a) one or more lytic peptides, which is not lysozyme, in combination with;
(b) one or more chitinases; and/or
(c) one or more β-1,3-glucanases
in a synergistically amount.

19. The plant of claim 18 wherein the lytic peptide is any natural or synthetic peptide or any functional derivative thereof that is capable of anti-pathogenically effective activity due to its ability to penetrate, lyse or otherwise impair the pathogen's cell membrane.

20. The plant of claim 19 wherein the lytic peptide is selected from the group consisting of mammalian defensins, cecropins, thionins, mellitins, insect defensins, magainins, attacins, dipteris, sapecins, cacrutins, xenopsins, or hybrids thereof.

21. The plant according to any one of claims 18 to 20 which is a maize plant.

22. A seed of a plant according to any one of claims 18 to 21 comprising recombinant DNA sequences as defined in any one of claims 18 to 20.

23. A method of preparing a transgenic plant which is able to synthesize one or more lytic peptides together with one or more chitinases; and/or one or more β-1,3-glucanases in a synergistically effective amount;
said method comprising the steps of
preparing by transformation and regeneration a transgenic plant comprising recombinant DNA sequences encoding one or more lytic peptides, which is not lysozyme together with one or more chitinases; and/or one or more β-1,3-glucanases.

24. A method of preparing a transgenic plant which is able to synthesize one or more lytic peptides, which is not lysozyme together with one or more chitinases; and/or one or more β-1,3-glucanases in a synergistically effective amount;
said method comprising the steps of
preparing by transformation and regeneration two or more transgenic plants comprising recombinant DNA sequences encoding one or more lytic peptides together with one or more chitinases; and/or one or more β-1,3-glucanases and crossing said plants using conventional breeding techniques.

## Claims (Claims for the following Contracting State(s): ES)

1. An anti-pathogenic composition for controlling plant pathogens comprising an active ingredient together with one or more agriculturally acceptable carriers; wherein the active ingredient comprises
(a) one or more lytic peptides, which is not lysozyme, in combination with;
(b) one or more chitinases; and/or
(c) one or more β-1,3-glucanases
(d) in a synergistically effective amount.

2. The composition of claim 1 wherein the lytic peptide is any natural or synthetic peptide or any functional derivative thereof that is capable of anti-pathogenically effective activity due to its ability to penetrate, lyse or otherwise impair the pathogen's cell membrane.

3. The composition of claim 2 wherein the lytic peptide is selected from the group consisting of mammalian defensins, cecropins, thionins, mellitins, insect defensins, magainins, attacins, dipteris, sapecins, cacrutins, xenopsins, or hybrids thereof.

4. The composition of any one of claims 1 to 3 wherein chitinase is present in the active ingredient at a relative concentration of about 1/10 c.

5. The composition of any one of claims 1 to 3 wherein chitinase is present in the active ingredient at a relative concentration of about 1/100 c.

6. The composition of any one of claims 1 to 3 wherein the β-1,3-glucanase is present in the active ingredient at a relative concentration of about 1/10 c.

7. The composition of any one of claims 1 to 3 wherein the β-1,3-glucanase is present in the active ingredient at a relative concentration of about 1/100 c.

8. The composition of any one of claims 1 to 3 wherein chitinase is present in the active ingredient at a relative concentration of about 1/10 c and the β-1,3-glucanase is present in the active ingredient at a relative concentration of about 1/10 c.

9. The composition of any one of claims 1 to 3 wherein chitinase is present in the active ingredient at a relative concentration of about 1/10 c and the β-1,3-glucanase is present in the active ingredient at a relative concentration of about 1/100 c.

10. The composition of any one of claims 1 to 3 wherein chitinase is present in the active ingredient at a relative concentration of about 1/100 c and the β-1,3-glucanase is present in the active ingredient at a relative concentration of about 1/100 c.

11. The composition of any one of claims 1 to 3 wherein chitinase is present in the active ingredient at a relative concentration of about 1/100 c and the β-1,3-glucanase is present in the active ingredient at a relative concentration of about 1/10 c.

12. The composition of claim 1, wherein one of the lytic peptides is synthetic peptide no. 3.

13. The composition of any one of claims 1 to 3 wherein the lytic peptides are present in a concentration from about 1 to about 200 ppm.

14. A method of controlling plant pathogens comprising applying to the pathogen or the locus thereof an anti-pathogenically effective amount of a composition comprising an active ingredient together with one or more agriculturally acceptable carriers; wherein the active ingredient comprises
(a) one or more lytic peptides which is not lysozyme, in combination with;
(b) one or more chitinases; and/or
(c) one or more β-1,3-glucanases
in a synergistically effective amount.

15. The method according to claim 14, wherein the lytic peptide is used in combination with one or more activated chitinases; and/or one or more activated β-1,3-glucanases, which are already present in the plant to be protected using an external inducing stimulus.

16. A method of controlling plant pathogens by expressing in a transgenic plant one or more lytic peptides together with one or more chitinases; and/or one or more β-1,3-glucanases in a synergistically effective amount;
said method comprising the steps of
(a) preparing a transgenic plant comprising recombinant DNA sequences encoding one or more lytic peptides, which is not lysozyme, one or more chitinases; and/or one or more β-1,3-glucanases; and
(b) causing the transgenic plant to synthesize the compounds mentioned in step (a).

17. The method according to claim 16, wherein the chitinase(s) and/or the β-1,3-glucanase(s) are part of an inducible expression system comprising an inducible gene and an inducing regulator and wherein the expression of at least one lytic peptide is followed by treatment with an inducing regulator which induces the accumulation of at least one natural or foreign chitinase and/or one natural or foreign β-1,3-glucanase.

18. A method of preparing a transgenic plant and seed thereof, which is able to synthesize one or more lytic peptides together with one or more chitinases; and/or one or more β-1,3-glucanases in a synergistically effective amount;
said method comprising the steps of
preparing by transformation and regeneration a transgenic plant comprising recombinant DNA sequences encoding one or more lytic peptides, which is not lysozyme together with one or more chitinases; and/or one or more β-1,3-glucanases.

19. A method of preparing a transgenic plant and seed thereof, which is able to synthesize one or more lytic peptides, which is not lysozyme together with one or more chitinases; and/or one or more β-1,3-glucanases in a synergistically effective amount;
said method comprising the steps of
preparing by transformation and regeneration two or more transgenic plants comprising recombinant DNA sequences encoding one or more lytic peptides together with one or more chitinases; and/or one or more β-1,3-glucanases and crossing said plants using conventional breeding techniques.

20. The method according to claim 18 or 19, wherein the plant is a maize plant.

## Patentansprüche (Patentansprüche für folgende(n) Vertragsstaat(en): AT, BE, CH, DE, DK, FR, GB, GR, IT, LU, NL, SE)

1. Antipathogene Zusammensetzung zur Bekämpfung von Pflanzenpathogenen, die einen wirksamen Bestandteil zusammen mit einem oder mehreren landwirtschaftlich akzeptablen Trägem umfaßt, wobei der wirksame Bestandteil
(a) ein oder mehrere lytische Peptide, bei denen es sich nicht um Lysozym handelt, in Kombination mit
(b) einer oder mehreren Chitinasen und/oder
(c) einer oder mehreren β-1,3-Glucanasen
in einer synergistisch wirksamen Menge umfaßt.

2. Zusammensetzung nach Anspruch 1, wobei das lytische Peptid ein beliebiges natürliches oder synthetisches Peptid oder ein beliebiges funktionelles Derivat hiervon mit der Fähigkeit ist, infolge seiner Fähigkeit, in die Zellmembran des Pathogens einzudringen, die Zellmembran des Pathogens zu lysieren oder in anderer Weise zu beeinträchtigen, eine antipathogen wirksame Aktivität zu entfalten.

3. Zusammensetzung nach Anspruch 2, wobei das lytische Peptid aus aus Säugetieren stammenden Defensinen, Cecropinen, Thioninen, Mellitinen, aus Insekten stammenden Defensinen, Magaininen, Attacinen, Dipterinen, Sapecinen, Cacrutinen, Xenopsinen oder Hybriden hiervon ausgewählt ist.

4. Zusammensetzung nach einem der Ansprüche 1 bis 3, wobei die Chitinase in dem wirksamen Bestandteil in einer relativen Konzentration von etwa 1/10 c vorhanden ist.

5. Zusammensetzung nach einem der Ansprüche 1 bis 3, wobei die Chitinase in dem wirksamen Bestandteil in einer relativen Konzentration von etwa 1/100 c vorhanden ist.

6. Zusammensetzung nach einem der Ansprüche 1 bis 3, wobei die β-1,3-Glucanase in dem wirksamen Bestandteil in einer relativen Konzentration von etwa 1/10 c vorhanden ist.

7. Zusammensetzung nach einem der Ansprüche 1 bis 3, wobei die β-1,3-Glucanase in dem wirksamen Bestandteil in einer relativen Konzentration von etwa 1/100 c vorhanden ist.

8. Zusammensetzung nach einem der Ansprüche 1 bis 3, wobei die Chitinase in dem wirksamen Bestandteil in einer relativen Konzentration von etwa 1/10 c und die β-1,3-Glucanase in dem wirksamen Bestandteil in einer relativen Konzentration von etwa 1/10 c vorhanden sind.

9. Zusammensetzung nach einem der Ansprüche 1 bis 3, wobei die Chitinase in dem wirksamen Bestandteil in einer relativen Konzentration von etwa 1/10 c und die β-1,3-Glucanase in dem wirksamen Bestandteil in einer relativen Konzentration von etwa 1/100 c vorhanden sind.

10. Zusammensetzung nach einem der Ansprüche 1 bis 3, wobei die Chitinase in dem wirksamen Bestandteil in einer relativen Konzentration von etwa 1/100 c und die β-1,3-Glucanase in dem wirksamen Bestandteil in einer relativen Konzentration von etwa 1/100 c vorhanden sind.

11. Zusammensetzung nach einem der Ansprüche 1 bis 3, wobei die Chitinase in dem wirksamen Bestandteil in einer relativen Konzentration von etwa 1/100 c und die β-1,3-Glucanase in dem wirksamen Bestandteil in einer relativen Konzentration von etwa 1/10 c vorhanden sind.

12. Zusammensetzung nach Anspruch 1, wobei eines der lytischen Peptide das synthetische Peptid Nr. 3 ist.

13. Zusammensetzung nach einem der Ansprüche 1 bis 3, wobei die lytischen Peptide in einer Konzentration von etwa 1 bis etwa 200 ppm vorhanden sind.

14. Verfahren zur Bekämpfung von Pflanzenpathogen durch Applizieren einer antipathogen wirksamen Menge einer Zusammensetzung, die einen wirksamen Bestandteil zusammen mit einem oder mehreren landwirtschaftlichen akzeptablen Trägem umfaßt, auf das Pathogen oder den Ort hiervon, wobei der wirksame Bestandteil
(a) ein oder mehrere lytische Peptide, bei denen es sich nicht um Lysozym handelt, in Kombination mit
(b) einer oder mehreren Chitinasen und/oder
(c) einer oder mehreren β-1,3-Glucanasen
in einer synergistisch wirksamen Menge umfaßt.

15. Verfahren nach Anspruch 14, wobei das lytische Peptid in Kombination mit einer oder mehreren aktivierten Chitinasen und/oder einer oder mehreren aktivierten β-1,3-Glucanasen, die bereits in der zu schützenden Pflanze unter Verwendung eines externen induzierenden Stimulus vorhanden sind, verwendet wird.

16. Verfahren zur Bekämpfung von Pflanzenpathogenen durch Exprimieren eines oder mehrerer lytischer Peptide zusammen mit einer oder mehreren Chitinasen und/oder einer oder mehreren β-1,3-Glucanasen in einer synergistisch wirksamen Menge in einer transgenen Pflanze, wobei das Verfahren die folgenden Stufen umfaßt:
(a) Herstellen einer transgenen Pflanze, die rekombinante DNA-Sequenzen mit Codierung für ein oder mehrere lytische Peptide, bei denen es sich nicht um Lysozym handelt, eine oder mehrere Chitinasen und/oder eine oder mehrere β-1,3-Glucanasen umfaßt, und
(b) Bewirken, daß die transgene Pflanze die in Stufe (a) erwähnten Verbindungen synthetisiert.

17. Verfahren nach Anspruch 16, wobei die Chitinase(n) und/oder die β-1,3-Glucanase(n) Teil eines induzierbaren Expressionssystems sind, das ein induzierbares Gen und einen induzierenden Regulator umfaßt, und wobei der Expression mindestens eines lytischen Peptids eine Behandlung mit einem induzierenden Regulator folgt, der die Anhäufung mindestens einer natürlichen oder fremden Chitinase und/oder einer natürlichen oder fremden β-1,3-Glucanase induziert.

18. Transgene Pflanze, die rekombinante DNA-Sequenzen mit Codierung für
(a) ein oder mehrere lytische Peptide, bei denen es sich nicht um Lysozym handelt, in Kombination mit
(b) einer oder mehreren Chitinasen und/oder
(c) einer oder mehreren β-1,3-Glucanasen
in einer synergistisch wirksamen Menge umfaßt.

19. Pflanze nach Anspruch 18, wobei das lytische Peptid ein beliebiges natürliches oder synthetisches Peptid oder ein beliebiges funktionelles Derivat hiervon ist, das infolge seiner Fähigkeit, in die Zellmembran des Pathogens einzudringen, die Zellmembran des Pathogens zu lysieren oder in anderer Weise zu beeinträchtigen, eine antipathogen wirksame Aktivität zu entfalten vermag.

20. Pflanze nach Anspruch 19, wobei das lytische Peptid aus aus Säugetieren stammenden Defensinen, Cecropinen, Thioninen, Mellitinen, aus Insekten stammenden Defensinen, Magaininen, Attacinen, Dipterinen, Sapecinen, Cacrutinen, Xenopsinen oder Hybriden hiervon ausgewählt ist.

21. Pflanze nach einem der Ansprüche 18 bis 20, bei der es sich um eine Maispflanze handelt.

22. Samen einer Pflanze nach einem der Ansprüche 18 bis 21, der rekombinante DNA-Sequenzen gemäß Definition nach einem der Ansprüche 18 bis 20 umfaßt.

23. Verfahren zur Herstellung einer transgenen Pflanze mit der Fähigkeit zur Synthese eines oder mehrerer lytischer Peptide zusammen mit einer oder mehreren Chitinasen und/oder einer oder mehreren β-1,3-Glucanasen in einer synergistisch wirksamen Menge, wobei das Verfahren die folgenden Stufen umfaßt:
Herstellen einer transgenen Pflanze durch Transformation und Regeneration, wobei die transgene Pflanze rekombinante DNA-Sequenzen mit Codierung für ein oder mehrere lytische Peptide, bei denen es sich nicht um Lysozym handelt, zusammen mit einer oder mehreren Chitinasen und/oder einer oder mehreren β-1,3-Glucanasen umfaßt.

24. Verfahren zur Herstellung einer transgenen Pflanze mit der Fähigkeit zur Synthese eines oder mehrerer lytischer Peptide, bei denen es sich nicht um Lysozym handelt, zusammen mit einer oder mehreren Chitinasen und/oder einer oder mehreren β-1,3-Glucanasen in einer synergistisch wirksamen Menge, wobei das Verfahren die folgenden Stufen umfaßt:
Herstellen von zwei oder mehr transgenen Pflanzen durch Transformation und Regeneration, wobei die transgenen Pflanzen rekombinante DNA-Sequenzen mit Codierung für ein oder mehrere lytische Peptide zusammen mit einer oder mehreren Chitinasen und/oder einer oder mehreren β-1,3-Glucanasen umfassen, und Kreuzen der Pflanzen unter Verwendung herkömmlicher Züchtungstechniken.

## Patentansprüche (Patentansprüche für folgende(n) Vertragsstaat(en): ES)

1. Antipathogene Zusammensetzung zur Bekämpfung von Pflanzenpathogenen, die einen wirksamen Bestandteil zusammen mit einem oder mehreren landwirtschaftlich akzeptablen Trägern umfaßt, wobei der wirksame Bestandteil
(a) ein oder mehrere lytische Peptide, bei denen es sich nicht um Lysozym handelt, in Kombination mit
(b) einer oder mehreren Chitinasen und/oder
(c) einer oder mehreren β-1,3-Glucanasen in einer synergistisch wirksamen Menge umfaßt.

2. Zusammensetzung nach Anspruch 1, wobei das lytische Peptid ein beliebiges natürliches oder synthetisches Peptid oder ein beliebiges funktionelles Derivat hiervon mit der Fähigkeit ist, infolge seiner Fähigkeit, in die Zellmembran des Pathogens einzudringen, die Zellmembran des Pathogens zu lysieren oder in anderer Weise zu beeinträchtigen, eine antipathogen wirksame Aktivität zu entfalten.

3. Zusammensetzung nach Anspruch 2, wobei das lytische Peptid aus aus Säugetieren stammenden Defensinen, Cecropinen, Thioninen, Mellitinen, aus Insekten stammenden Defensinen, Magaininen, Attacinen, Dipterinen, Sapecinen, Cacrutinen, Xenopsinen oder Hybriden hiervon ausgewählt ist.

4. Zusammensetzung nach einem der Ansprüche 1 bis 3, wobei die Chitinase in dem wirksamen Bestandteil in einer relativen Konzentration von etwa 1/10 c vorhanden ist.

5. Zusammensetzung nach einem der Ansprüche 1 bis 3, wobei die Chitinase in dem wirksamen Bestandteil in einer relativen Konzentration von etwa 1/100 c vorhanden ist.

6. Zusammensetzung nach einem der Ansprüche 1 bis 3, wobei die β-1,3-Glucanase in dem wirksamen Bestandteil in einer relativen Konzentration von etwa 1/10 c vorhanden ist.

7. Zusammensetzung nach einem der Ansprüche 1 bis 3, wobei die β-1,3-Glucanase in dem wirksamen Bestandteil in einer relativen Konzentration von etwa 1/100 c vorhanden ist.

8. Zusammensetzung nach einem der Ansprüche 1 bis 3, wobei die Chitinase in dem wirksamen Bestandteil in einer relativen Konzentration von etwa 1/10 c und die β-1,3-Glucanase in dem wirksamen Bestandteil in einer relativen Konzentration von etwa 1/10 c vorhanden sind.

9. Zusammensetzung nach einem der Ansprüche 1 bis 3, wobei die Chitinase in dem wirksamen Bestandteil in einer relativen Konzentration von etwa 1/10 c und die β-1.3-Glucanase in dem wirksamen Bestandteil in einer relativen Konzentration von etwa 1/100 c vorhanden sind.

10. Zusammensetzung nach einem der Ansprüche 1 bis 3, wobei die Chitinase in dem wirksamen Bestandteil in einer relativen Konzentration von etwa 1/100 c und die β-1,3-Glucanase in dem wirksamen Bestandteil in einer relativen Konzentration von etwa 1/100 c vorhanden sind.

11. Zusammensetzung nach einem der Ansprüche 1 bis 3, wobei die Chitinase in dem wirksamen Bestandteil in einer relativen Konzentration von etwa 1/100 c und die β-1,3-Glucanase in dem wirksamen Bestandteil in einer relativen Konzentration von etwa 1/10 c vorhanden sind.

12. Zusammensetzung nach Anspruch 1, wobei eines der lytischen Peptide das synthetische Peptid Nr. 3 ist.

13. Zusammensetzung nach einem der Ansprüche 1 bis 3, wobei die lytischen Peptide in einer Konzentration von etwa 1 bis etwa 200 ppm vorhanden sind.

14. Verfahren zur Bekämpfung von Pflanzenpathogen durch Applizieren einer antipathogen wirksamen Menge einer Zusammensetzung, die einen wirksamen Bestandteil zusammen mit einem oder mehreren landwirtschaftlichen akzeptablen Trägem umfaßt, auf das Pathogen oder den Ort hiervon, wobei der wirksame Bestandteil
(a) ein oder mehrere lytische Peptide, bei denen es sich nicht um Lysozym handelt, in Kombination mit
(b) einer oder mehreren Chitinasen und/oder
(c) einer oder mehreren β-1,3-Glucanasen
in einer synergistisch wirksamen Menge umfaßt.

15. Verfahren nach Anspruch 14, wobei das lytische Peptid in Kombination mit einer oder mehreren aktivierten Chitinasen und/oder einer oder mehreren aktivierten β-1,3-Glucanasen, die bereits in der zu schützenden Pflanze unter Verwendung eines externen induzierenden Stimulus vorhanden sind. verwendet wird.

16. Verfahren zur Bekämpfung von Pflanzenpathogenen durch Exprimieren eines oder mehrerer lytischer Peptide zusammen mit einer oder mehreren Chitinasen und/oder einer oder mehreren β-1,3-Glucanasen in einer synergistisch wirksamen Menge in einer transgenen Pflanze, wobei das Verfahren die folgenden Stufen umfaßt:
(a) Herstellen einer transgenen Pflanze, die rekombinante DNA-Sequenzen mit Codierung für ein oder mehrere lytische Peptide, bei denen es sich nicht um Lysozym handelt, eine oder mehrere Chitinasen und/oder eine oder mehrere β-1,3-Glucanasen umfaßt, und
(b) Bewirken, daß die transgene Pflanze die in Stufe (a) erwähnten Verbindungen synthetisiert.

17. Verfahren nach Anspruch 16, wobei die Chitinase(n) und/oder die β-1,3-Glucanase(n) Teil eines induzierbaren Expressionssystems sind, das ein induzierbares Gen und einen induzierenden Regulator umfaßt, und wobei der Expression mindestens eines lytischen Peptids eine Behandlung mit einem induzierenden Regulator folgt, der die Anhäufung mindestens einer natürlichen oder fremden Chitinase und/oder einer natürlichen oder fremden β-1,3-Glucanase induziert.

18. Verfahren zur Herstellung einer transgenen Pflanze und eines Samens hiervon mit der Fähigkeit zur Synthese eines oder mehrerer lytischer Peptide zusammen mit einer oder mehreren Chitinasen und/oder einer oder mehreren β-1,3-Glucanasen in einer synergistisch wirksamen Menge, wobei das Verfahren die folgenden Stufen umfaßt:
Herstellen einer transgenen Pflanze durch Transformation und Regeneration, wobei die transgene Pflanze rekombinante DNA-Sequenzen mit Codierung für ein oder mehrere lytische Peptide, bei denen es sich nicht um Lysozym handelt, zusammen mit einer oder mehreren Chitinasen und/oder einer oder mehreren ß-1,3-Glucanasen umfaßt.

19. Verfahren zur Herstellung einer transgenen Pflanze und eines Samens hiervon mit der Fähigkeit zur Synthese eines oder mehrerer lytischer Peptide, bei denen es sich nicht um Lysozym handelt, zusammen mit einer oder mehreren Chitinasen und/oder einer oder mehreren β-1,3-Glucanasen in einer synergistisch wirksamen Menge, wobei das Verfahren die folgenden Stufen umfaßt:
Herstellen von zwei oder mehr transgenen Pflanzen durch Transformation und Regeneration. wobei die transgenen Pflanzen rekombinante DNA-Sequenzen mit Codierung für ein oder mehrere lytische Peptide zusammen mit einer oder mehreren Chitinasen und/oder einer oder mehreren β-1,3-Glucanasen umfassen, und Kreuzen der Pflanzen unter Verwendung herkömmlicher Züchtungstechniken.

20. Verfahren nach Anspruch 18 oder 19, wobei es sich der der Pflanze um eine Maispflanze handelt.

## Revendications (Revendications pour l'(les) Etat(s) contractant(s) suivant(s): AT, BE, CH, DE, DK, GB, GR, IT, LU, NL, SE)

1. Composition antipathogène permettant de lutter contre les phytopathogènes, comprenant un ingrédient actif en conjonction avec un ou plusieurs agents acceptables en agriculture, dans laquelle l'ingrédient actif comprend
(a) un ou plusieurs peptides de lyse, qui ne soient pas un lysozyme, en combinaison avec:
(b) une ou plusieurs chitinases; et/ou
(c) une ou plusieurs β-1,3-glucanases
en quantité efficace en synergie.

2. Composition selon la revendication 1, dans laquelle le peptide lytique est un quelconque peptide naturel ou synthétique ou un quelconque dérivé fonctionnel de ce peptide, qui est doué d'une activité efficace en tant qu'antipathogène en raison de sa capacité à pénétrer, lyser ou altérer d'une autre façon la membrane cellulaire d'un pathogène.

3. Composition selon la revendication 2, dans lequel le peptide de lyse est choisi dans le groupe constitué par les défensines de mammifères, les cécropines, les thionines, les mellitines, les défensines d'insectes, les magaïnines, les attacines, le dipteris, les sapécines, les cacrutines, les xénopsines, ou leurs hybrides.

4. Composition selon l'une quelconque des revendications 1 à 3, dans laquelle la chitinase est présente dans l'ingrédient actif à une concentration relative d'environ 1/10 c.

5. Composition selon l'une quelconque des revendications 1 à 3, dans laquelle la chitinase est présente dans l'ingrédient actif à une concentration relative d'environ 1/100 c.

6. Composition selon l'une quelconque des revendications 1 à 3, dans laquelle la β-1,3-glucanase est présente dans l'ingrédient actif à une concentration relative d'environ 1/10 c.

7. Composition selon l'une quelconque des revendications 1 à 3, dans laquelle la β-1,3-glucanase est présente dans l'ingrédient actif à une concentration relative d'environ 1/100 c.

8. Composition selon l'une quelconque des revendications 1 à 3, dans laquelle la chitinase est présente dans l'ingrédient actif à une concentration relative d'environ 1/10 c et la β-1,3-glucanase est présente dans l'ingrédient actif à une concentration relative d'environ 1/10 c.

9. Composition selon l'une quelconque des revendications 1 à 3, dans laquelle la chitinase est présente dans l'ingrédient actif à une concentration relative d'environ 1/10 c et la β-1,3-glucanase est présente dans l'ingrédient actif à une concentration relative d'environ 1/100 c.

10. Composition selon l'une quelconque des revendications 1 à 3, dans laquelle la chitinase est présente dans l'ingrédient actif à une concentration relative d'environ 1/100 c et la β-1,3-glucanase est présente dans l'ingrédient actif à une concentration relative d'environ 1/100 c.

11. Composition selon l'une quelconque des revendications 1 à 3, dans laquelle la chitinase est présente dans l'ingrédient actif à une concentration relative d'environ 1/100 c et la β-1,3-glucanase est présente dans l'ingrédient actif à une concentration relative d'environ 1/10 c.

12. Composition selon la revendication 1, dans laquelle l'un des peptides de lyse est le peptide synthétique n° 3.

13. Composition selon l'une quelconque des revendications 1 à 3, dans laquelle les peptides de lyse sont présents à une concentration d'environ 1 à environ 200 ppm.

14. Procédé de lutte contre les phytopathogènes comprenant l'application au pathogène ou son locus d'une quantité efficace antipathogène d'une composition comprenant un ingrédient actif en même temps qu'un ou plusieurs agents acceptables en agriculture, dans laquelle l'ingrédient actif comprend
(a) un ou plusieurs peptides de lyse, qui ne soient pas un lysozyme, en combinaison avec:
(b) une ou plusieurs chitinases; et/ou
(c) une ou plusieurs β-1,3-glücanases
en quantité efficace en synergie.

15. Procédé selon la revendication 14, dans lequel le peptide de lyse est employé en combinaison avec une ou plusieurs chitinases activées; et/ou une ou plusieurs β-1,3-glucanases activées, qui sont déjà présentes dans la plante à protéger au moyen d'un stimulus d'induction externe.

16. Procédé pour lutter contre des phytopathogènes en exprimant dans une plante transgénique un ou plusieurs peptides de lyse ainsi qu'une ou plusieurs chitinascs, et/ou une ou plusieurs β-1,3-glucanases, en quantité efficace en synergie, ledit procédé comprenant les étapes consistant à
(a) préparer une plante transgénique comprenant des séquences d'ADN recombinant codant pour un ou plusieurs peptides de lyse, qui ne soient pas un lysozyme, une ou plusieurs chitinases et/ou une ou plusieurs β-1,3-glucanases; et
(b) faire en sorte que la plante transgénique synthétise les composés mentionnés dans l'étape (a).

17. Procédé selon la revendication 16, dans lequel la ou les chitanases et/ou le ou les β-1,3-glucanases font partie d'un système d'expression inductible comprenant un gène inductible et un régulateur inducteur et dans lequel l'expression d'au moins un peptide de lyse est suivie par un traitement avec un régulateur inducteur qui induit l'accumulation d'au moins une chitinase naturelle ou étrangère et/ou une β-1,3-glucanase naturelle ou étrangère.

18. Plante transgénique comprenant des séquences d'ADN recombinant codant pour
(a) un ou plusieurs peptides de lyse, qui ne soient pas un lysozyme, en combinaison avec:
(b) une ou plusieurs chitinases; et/ou
(c) une ou plusieurs β-1,3-glucanases
en quantité synergique.

19. Plante selon la revendication 18, dans laquelle le peptide lytique est un quelconque peptide naturel ou synthétique ou un quelconque dérivé fonctionnel de ce peptide, qui est doué d'une activité efficace en tant qu'antipathogène en raison de sa capacité à pénétrer, lyser ou altérer d'une autre façon la membrane cellulaire d'un pathogène.

20. Plante selon la revendication 19, dans laquelle le peptide de lyse est choisi dans le groupe constitué par les défensines de mammifères, les cécropines, les thionines, les mellitines, les défensines d'insectes, les magaïnines, les attacines, le dipteris, les sapécines, les cacrutines, les xénopsines, ou leurs hybrides.

21. Plante selon l'une quelconque des revendications 18 à 20, qui est un plant de maïs.

22. Graine d'une plante selon l'une quelconque des revendications 18 à 21 comprenant des séquences d'ADN recombinant telles que définies dans l'une quelconque des revendications 18 à 20.

23. Procédé de préparation d'une plante transgénique capable de synthétiser un ou plusieurs peptides de lyse ainsi qu'une ou plusieurs chitinases, et/ou une ou plusieurs β-1,3-glucanases, en quantité efficace en synergie, ledit procédé comprenant les étapes consistant à
préparer par transformation et régénération une plante transgénique comprenant des séquences d'ADN recombinant codant pour un ou plusieurs peptides de lyse, qui ne soient pas un lysozyme, ainsi qu'une ou plusieurs chitinases et/ou une ou plusieurs β-1,3-glucanases.

24. Procédé de préparation d'une plante transgénique capable de synthétiser un ou plusieurs peptides de lyse, qui ne soient pas un lysozyme, ainsi qu'une ou plusieurs chitinases et/ou une ou plusieurs β-1,3-glucanases en quantité efficace en synergie, ledit procédé comprenant les étapes consistant à
préparer par transformation et régénération au moins deux plantes transgéniques comprenant des séquences d'ADN recombinant codant pour un ou plusieurs peptides de lyse ainsi qu'une ou plusieurs chitinases et/ou une ou plusieurs β-1,3-glucanases et croiser lesdites plantes en utilisant des techniques de reproduction classiques.

## Revendications (Revendications pour l'(les) Etat(s) contractant(s) suivant(s): ES)

1. Composition antipathogène permettant de lutter contre les phytopathogènes, comprenant un ingrédient actif en même temps qu'un ou plusieurs agents acceptables en agriculture, dans laquelle l'ingrédient actif comprend
(a) un ou plusieurs peptides de lyse, qui ne soient pas un lysozyme, en combinaison avec:
(b) une ou plusieurs chitinases; et/ou
(c) une ou plusieurs β-1,3-glucanases
(d) en quantité efficace en synergie.

2. Composition selon la revendication 1, dans laquelle le peptide lytique est un quelconque peptide naturel ou synthétique ou un quelconque dérivé fonctionnel de ce peptide, qui est doué d'une activité efficace en tant qu'antipathogène en raison de sa capacité à pénétrer, lyser ou endommager d'une autre façon la membrane cellulaire d'un pathogène.

3. Composition selon la revendication 2, dans lequel le peptide de lyse est choisi dans le groupe constitué par les défensines de mammifères, les cécropines, les thionines, les mellitincs, les défensines d'insectes, les magaïnines, les attacines, le dipteris, les sapécines, les cacrutines, les xénopsines, ou leurs hybrides.

4. Composition selon l'une quelconque des revendications 1 à 3, dans laquelle la chitinase est présente dans l'ingrédient actif à une concentration relative d'environ 1/10 c.

5. Composition selon l'une quelconque des revendications 1 à 3, dans laquelle la chitinase est présente dans l'ingrédient actif à une concentration relative d'environ 1/100 c.

6. Composition selon l'une quelconque des revendications 1 à 3, dans laquelle la β-1,3-glucanase est présente dans l'ingrédient actif à une concentration relative d'environ 1/10 c.

7. Composition selon l'une quelconque des revendications 1 à 3, dans laquelle la β-1,3-glucanase est présente dans l'ingrédient actif à unc concentration relative d'environ 1/100 c.

8. Composition selon l'une quelconque des revendications 1 à 3, dans laquelle la chitinase est présente dans l'ingrédient actif à une concentration relative d'environ 1/10 c et la β-1,3-glucanase est présente dans l'ingrédient actif à une concentration relative d'environ 1/10 c.

9. Composition selon l'une quelconque des revendications 1 à 3, dans laquelle la chitinase est présente dans l'ingrédient actif à une concentration relative d'environ 1/10 c et la β-1,3-glucanase est présente dans l'ingrédient actif à une concentration relative d'environ 1/100 c.

10. Composition selon l'une quelconque des revendications 1 à 3, dans laquelle la chitinase est présente dans l'ingrédient actif à une concentration relative d'environ 1/100 c et la β-1,3-glucanase est présente dans l'ingrédient actif à une concentration relative d'environ 1/100 c.

11. Composition selon l'une quelconque des revendications 1 à 3, dans laquelle la chitinase est présente dans l'ingrédient actif à une concentration relative d'environ 1/100 c et la β-1,3-glucanase est présente dans l'ingrédient actif à une concentration relative d'environ 1/10 c.

12. Composition selon la revendication 1, dans laquelle l'un des peptides de lyse est le peptide synthétique n° 3.

13. Composition selon l'une quelconque des revendications 1 à 3, dans laquelle les peptides de lyse sont présents à une concentration d'environ 1 à environ 200 ppm.

14. Procédé de lutte contre les phytopathogènes comprenant l'application au pathogène ou son locus d'une quantité efficace antipathogène d'une composition comprenant un ingrédient actif en même temps qu'un ou plusieurs agents acceptables en agriculture, dans laquelle l'ingrédient actif comprend
(a) un ou plusieurs peptides de lyse, qui ne soient pas un lysozyme, en combinaison avec:
(b) une ou plusieurs chitinases; et/ou
(c) une ou plusieurs β-1,3-glucanases
en quantité efficace en synergie.

15. Procédé selon la revendication 14, dans lequel le peptide de lyse est employé en combinaison avec une ou plusieurs chitinases activées, et/ou une ou plusieurs β-1,3-glueanases activées, qui sont déjà présentes dans la plante à protéger au moyen d'un stimulus d'induction externe,

16. Procédé pour lutter contre des phytopathogènes en exprimant dans une plante transgénique un ou plusieurs peptides de lyse ainsi qu'une ou plusieurs chitinases, et/ou une ou plusieurs β-1,3-glucanases, en quantité efficace en synergie, ledit procédé comprenant les étapes consistant à
(c) préparer une plante transgénique comprenant des séquences d'ADN recombinant codant pour un ou plusieurs peptides de lyse, qui ne soient pas un lysozyme, une ou plusieurs chitinases et/ou une ou plusieurs β-1,3-glucallascs; et
(d) faire en sorte que la plante transgéniquc synthétise les composés mentionnés dans l'étape (a).

17. Procédé selon la revendication 16, dans lequel la ou les chitanases et/ou le ou les β-1,3-glucanases font partie d'un système d'expression inductible comprenant un gène inductible et un régulateur inducteur et dans lequel l'expression d'au moins un peptide de lyse est suivie par un traitement avec un régulateur inducteur qui induit l'accumulation d'au moins une chitinase naturelle ou étrangère et/ou une β-1,3-glucanase naturelle ou étrangère,

18. Procédé de préparation d'une plante transgénique et de ses graines, qui soit capable de synthétiser un ou plusieurs peptides de lyse ainsi qu'une ou plusieurs chitinases, et/ou une ou plusieurs β-1,3-glucanases, en quantité efficace en synergie, ledit procédé comprenant les étapes consistant à
préparer par transformation et régénération une plante transgénique comprenant des séquences d'ADN recombinant codant pour un ou plusieurs peptides de lyse, qui ne soient pas un lysozyme, ainsi qu'une ou plusieurs chitinases et/ou une ou plusieurs β-1,3-glucanases.

19. Procédé de préparation d'une plante transgénique et de ses graines qui est capable de synthétiser un ou plusieurs peptides de lyse, qui ne soient pas un lysozyme, ainsi qu'une ou plusieurs chitinases et/ou une ou plusieurs β-1,3-glucanases en quantité efficace en synergie, ledit procédé comprenant les étapes consistant à
préparer par transformation et régénération au moins deux plantes transgéniques comprenant des séquences d'ADN recombinant codant pour un ou plusieurs peptides de lyse ainsi qu'une ou plusieurs chitinases et/ou une ou plusieurs β-1,3-glucanases, et croiser lesdites plantes en utilisant des techniques de reproduction classiques.

20. Procédé selon la revendication 18 ou 19, dans lequel la plante est un plant de maïs.
